# EUROPEAN PATENT APPLICATION

(11) **EP 4 365 283 A1**
(43) Date of publication of application: **08.05.2024**
(21) Application number: 22833299.5
(22) Date of filing: 30.06.2022
(51) Int. Cl.: C12N 5/0789, C12N 5/10, C12N 15/09, A61K 35/15, A61P 7/00

(54) **METHOD FOR IMPROVING PROLIFERATIVE PROPERTIES OF COMMON MYELOID PROGENITOR CELLS (CMP) OR MYELOCYTIC PROGENITOR CELLS**

(30) Priority: 30.06.2021 JP 2021109513
(71) Applicant: National University Corporation Chiba University, Chiba-shi, Chiba 263-8522 (JP); Kyoto University, Kyoto-shi, Kyoto 606-8501 (JP)
(72) Inventor: TAKAYAMA, Naoya, Chiba-shi, Chiba 260-8670 (JP); ETO, Koji, Kyoto-shi, Kyoto 606-8501 (JP); NAKAMURA, Sou, Kyoto-shi, Kyoto 606-8501 (JP); PAUL, Sudip Kumar, Chiba-shi, Chiba 260-8670 (JP)
(74) Representative: Weickmann & Weickmann PartmbB
(86) International application number: PCT/JP2022/026341
(87) International publication number: WO 2023/277153

(57) **Abstract**

The present invention relates to a method for improving proliferative property of a CMP or a myeloid progenitor cell, including a step of forcibly expressing a MYC family gene and BMI1 gene in any cell in the process of differentiation from a hematopoietic progenitor cell into a myeloid progenitor cell, wherein the myeloid progenitor cell is a progenitor cell of macrophage, dendritic cell, granulocyte, erythroblast, or erythrocyte.

## Description

### [Technical Field]

The present invention widely relates to a method for improving proliferative properties of common myeloid progenitor cells (CMP) or myeloid progenitor cells, and the like.

### [Background Art]

Peripheral blood cells have been differentiated from hematopoietic stem cells via hematopoietic progenitor cells of each lineage. Common myeloid progenitor cells are hematopoietic progenitor cells that produce platelets, erythrocytes, and non-lymphoid leukocyte cells (neutrophils, macrophages, basophils, dendritic cells, etc.). They are present in bone marrow in steady state, but differentiate as needed during trauma, infection, etc. to provide mature blood cells. Neutrophils, macrophages, basophils, dendritic cells, and the like produced by them are the main players in innate immunity and contribute to defense against various pathogens, elimination of tumors and degenerated autologous cells, allergic reactions, and acute and chronic inflammations.

These cells are expected to be a source for drug screening for inflammation and allergy and for cell therapy to remove foreign substances in the body. In conventional techniques, methods of inducing differentiation in vitro from cord blood, bone marrow blood, human ES/iPS cells, and the like have been used. However, all these techniques are complicated and difficult to prepare a large number of cells.

### [Citation List]

### [Non Patent Literature]

[Non Patent Literature 1]
   Jie Z, Zhang Y, Wang C, Shen B, Guan X, Ren Z, et al. Large-scale ex vivo generation of human neutrophils from cord blood CD34+ cells. PLoS One. 2017; 12(3).
[Non Patent Literature 2]
   Caux C, Vanbervliet B, Massacrier C, Dezutter-Dambuyant C, De Saint-Vis B, Jacquet C, et al. CD34+ hematopoietic progenitors from human cord blood differentiate along two independent dendritic cell pathways in response to GM-CSF+TNFα. J Exp Med. 1996; 184(2):695-706.
[Non Patent Literature 3]
   Lachmann N, Ackermann M, Frenzel E, Liebhaber S, Brennig S, Happle C, et al. Large-scale hematopoietic differentiation of human induced pluripotent stem cells provides granulocytes or macrophages for cell replacement therapies. Stem Cell Reports. 2015;
[Non Patent Literature 4]
   Hiramoto T, Ebihara Y, Mizoguchi Y, Nakamura K, Yamaguchi K, Ueno K, et al. Wnt3a stimulates maturation of impaired neutrophils developed from severe congenital neutropenia patient-derived pluripotent stem cells. Proc Natl Acad Sci U S A. 2013; 110(8):3023-8.
[Non Patent Literature 5]
   Sweeney CL, Teng R, Wang H, Merling RK, Lee J, Choi U, et al. Molecular Analysis of Neutrophil Differentiation from Human Induced Pluripotent Stem Cells Delineates the Kinetics of Key Regulators of Hematopoiesis. Stem Cells. 2016; 34(6):1513-26.
[Non Patent Literature 6]
   Takata K, Kozaki T, Lee CZW, Thion MS, Otsuka M, Lim S, et al. Induced-Pluripotent-Stem-Cell-Derived Primitive Macrophages Provide a Platform for Modeling Tissue-Resident Macrophage Differentiation and Function. Immunity. 2017; 47(1):183-198.e6.
[Non Patent Literature 7]
   Cao X, Yakala GK, van den Hil FE, Cochrane A, Mummery CL, Orlova V V. Differentiation and Functional Comparison of Monocytes and Macrophages from hiPSCs with Peripheral Blood Derivatives. Stem Cell Reports. 2019; 12(6):1282-97.
[Non Patent Literature 8]
   Ackermann M, Kempf H, Hetzel M, Hesse C, Hashtchin AR, Brinkert K, et al. Bioreactor-based mass production of human iPSC-derived macrophages enables immunotherapies against bacterial airway infections. Nat Commun. 2018; 9(1).

### [Summary of Invention]

### [Technical Problem]

In the method using hematopoietic progenitor cells derived from cord blood or bone marrow blood, neutrophils, macrophages, and the like can be obtained by adding growth factors (Non Patent Literatures 1, 2). However, proliferation of cord blood and bone marrow blood itself has a limit and the difference among lots is large and therefore, it is difficult to prepare large quantities of cells having uniform quality.

In the method using human ES/iPS cells, ES/iPS cells themselves can proliferate almost infinitely. However, the differentiation induction method is complicated, and the induction efficiency into blood cells is poor, making it difficult to supply massively at a clinically applicable level (Non Patent Literatures 3 to 7). For example, it is reported that macrophage differentiation from human iPS cells is limited to 10⁸ cells in a 250 mL culture system (Non Patent Literature 8). In conventional techniques, there is a significant shortage of cells for both drug screening and cell therapy, and the development of more efficient induction methods is essential.

As immortalized cell lines of myeloid lineage, there is a cell line that has been established at low probability by culture from leukemia patients. However, in the cell line, leukemia mutant gene is constantly expressed, normal differentiation is impossible, and drug screening using mature cells is impossible. Also, it cannot be used as a source of cell therapy due to the risk of canceration.

In view of such situation, the present invention aims to provide a novel method for improving the proliferation property of common myeloid progenitor (CMP) cells or myeloid progenitor cells, for the establishment of a stable production system for cells such as neutrophils, macrophages, basophils, and dendritic cells.

### [Solution to Problem]

The present inventors have conducted intensive studies in an attempt to solve the aforementioned problems and found that the proliferative property of CMPs or myeloid progenitor cells can be improved by forcibly expressing a MYC family gene and BMI1 gene in any cells in the process of differentiation from hematopoietic progenitor cells into specific myeloid progenitor cells, which resulted in the completion of the present invention.

Accordingly, the present invention encompasses the following inventions.
[1] A method for improving proliferative property of a CMP or a myeloid progenitor cell, comprising a step of forcibly expressing a MYC family gene and BMI1 gene in any cell in the process of differentiation from a hematopoietic progenitor cell into a myeloid progenitor cell, wherein
   the myeloid progenitor cell is a progenitor cell of macrophage, dendritic cell, granulocyte, erythroblast, or erythrocyte.
[2] The method of [1], further comprising a step of extracting the CMP or myeloid progenitor cell.
[3] The method of [1] or [2], further comprising a step of suppressing the expression of the MYC family gene and the BMI1 gene, or the function of an expression product thereof in the CMP or myeloid progenitor cell.
[4] The method of any of [1] to [3], further comprising a step of forcibly expressing BCL-XL gene in the CMP or myeloid progenitor cell.
[5] The method of [4], further comprising a step of suppressing the expression of the BCL-XL gene, or the function of an expression product thereof in the CMP or myeloid progenitor cell.
[6] The method of any of [1] to [5], further comprising a step of suppressing the expression of at least one of CDKN1A gene and p53 gene, or the function of an expression product thereof, in the CMP or myeloid progenitor cell.
[7] A method for producing a CMP or a myeloid progenitor cell, comprising a step of culturing the CMP or myeloid progenitor cell obtained by the method of any of [1] to [6].
[8] A method for producing a CMP-lineage differentiated cell, comprising a step of differentiating the CMP or myeloid progenitor cell obtained by the method of any of [1] to [7].
[9] The method of [8], wherein the CMP-lineage differentiated cell is macrophage, dendritic cell, granulocyte, erythroblast, or erythrocyte.
[10] A pharmaceutical composition comprising the CMP or myeloid progenitor cell obtained by the method of any of [1] to [7], or the CMP-lineage differentiated cell obtained by the method of [8] or [9].
[11] A method for treating or preventing a disease, comprising administering the CMP or myeloid progenitor cell obtained by the method of any of [1] to [7], the CMP-lineage differentiated cell obtained by the method of [8] or [9], or the pharmaceutical composition of [10] to a patient in need thereof.
[12] The CMP or myeloid progenitor cell obtained by the method of any of [1] to [7].
[13] The CMP-lineage differentiated cell obtained by the method of [8] or [9].
[14] A proliferation promoting agent of a CMP or a myeloid progenitor cell, comprising
   a molecule that forcibly expresses a MYC family gene and BMI1 gene as an active ingredient, wherein
   the myeloid progenitor cell is a progenitor cell of macrophage, dendritic cell, granulocyte, erythroblast, or erythrocyte.
[15] A CMP or a myeloid progenitor cell comprising a MYC family gene operably linked to a first exogenous promoter and BMI1 gene operably linked to a second exogenous promoter, wherein
   the myeloid progenitor cell is a progenitor cell of macrophage, dendritic cell, granulocyte, erythroblast, or erythrocyte.
[16] A cell population comprising the CMP or myeloid progenitor cell of [15] at a ratio of not less than 10% with respect to the whole cell population.
[17] A cell preparation comprising the cell population of [16].
[18] A method for producing macrophages, comprising the following steps:
   1) a step of forcibly expressing a MYC family gene and BMI1 gene in any cell in the process of differentiation from hematopoietic progenitor cells into macrophage progenitor cells,
   2) a step of culturing and proliferating the cells obtained in step 1,
   3) a step of suppressing forced expression of the MYC family gene and the BMI1 gene in the cells obtained in step 2 and further culturing the cells under macrophage differentiation conditions to promote differentiation into and maturation of macrophages.
[19] The method of claim 14, wherein the step 1 further comprises forcibly expressing BCL-XL gene in any cells in the process of differentiation from hematopoietic progenitor cells into macrophage progenitor cells.
[20] The method of [19], wherein the step 3 further comprises suppressing forced expression of the BCL-XL gene in the cells obtained in step 2.
[21] The method of any of [18] to [20], wherein the step 1 further comprises suppressing expression of CDKN1A gene and/or p53 gene, or the function of an expression product thereof in any cells in the process of differentiation from hematopoietic progenitor cells into macrophage progenitor cells.

[1B] A method for improving proliferative property of a CMP or a myeloid progenitor cell, comprising a step of forcibly expressing BCL-XL gene in the CMP or myeloid progenitor cell, wherein
   the myeloid progenitor cell is a GMP, a macrophage progenitor cell, or a dendritic cell progenitor cell.
[2B] The method of [1B], further comprising a step of extracting the CMP or myeloid progenitor cell.
[3B] The method of [1B] or [2B], further comprising a step of forcibly expressing a MYC family gene and BMI1 gene in the CMP or myeloid progenitor cell.
[4B] The method of any of [1B] to [3B], further comprising a step of suppressing the expression of at least one of CDKN1A gene and p53 gene, or the function of an expression product thereof, in the CMP or myeloid progenitor cell.
[5B] A method for producing CMPs or myeloid progenitor cells, comprising a step of culturing the CMPs or myeloid progenitor cells obtained by the method of any of [1B] to [4B].
[6B] A method for producing a CMP-lineage differentiated cell, comprising a step of differentiating the CMP or myeloid progenitor cell obtained by the method of any of [1B] to [5B].
[7B] The method of [6B], wherein the CMP-lineage differentiated cell is a macrophage or a dendritic cell.
[8B] A pharmaceutical composition comprising the CMP or myeloid progenitor cell obtained by the method of any of [1B] to [5B], or the CMP-lineage differentiated cell obtained by the method of [6B] or [7B].
[9B] A method for treating or preventing a disease, comprising administering the CMP or myeloid progenitor cell obtained by the method of any of [1B] to [5B], the CMP-lineage differentiated cell obtained by the method of [6B] or [7B], or the pharmaceutical composition of [8B] to a patient in need thereof.
[10B] The CMP or myeloid progenitor cell obtained by the method of any of [1B] to [5B].
[11B] The CMP-lineage differentiated cell obtained by the method of [6B] or [7B].
[12B] A proliferation promoting agent of CMP or myeloid progenitor cell, comprising
   a molecule that forcibly expresses BCL-XL gene as an active ingredient, wherein
   the myeloid progenitor cell is a GMP, a macrophage progenitor cell, or a dendritic cell progenitor cell.
[13B] A CMP or myeloid progenitor cell comprising BCL-XL gene operably linked to an exogenous promoter, wherein
   the myeloid progenitor cell is a GMP, a macrophage progenitor cell, or a dendritic cell progenitor cell.
[14B] A cell population comprising the CMP or myeloid progenitor cell of [13B] at a ratio of not less than 10% with respect to the whole cell population.
[15B] A cell preparation comprising the cell population of [14B] .

[1C] A method for improving proliferative property of a CMP or a myeloid progenitor cell, comprising a step of suppressing the expression of at least one of CDKN1A gene and p53 gene, or the function of an expression product thereof, in the CMP or myeloid progenitor cell, wherein
   the myeloid progenitor cell is a GMP, a macrophage progenitor cell, a dendritic cell progenitor cell, or an erythrocyte progenitor cell.
[2C] The method of [1C], further comprising a step of extracting the CMP or myeloid progenitor cell.
[3C] The method of [1C] or [2C], further comprising a step of forcibly expressing a MYC family gene and BMI1 gene in the CMP or myeloid progenitor cell.
[4C] The method of any of [1C] to [3C], further comprising a step of forcibly expressing BCL-XL gene in the CMP or myeloid progenitor cell.
[5C] A method for producing a CMP or a myeloid progenitor cell, comprising a step of culturing the CMP or myeloid progenitor cell obtained by the method of any of [1C] to [4C].
[6C] A method for producing a CMP-lineage differentiated cell, comprising a step of differentiating the CMP or myeloid progenitor cell obtained by the method of any of [1C] to [5C].
[7C] The method of [6C], wherein the CMP-lineage differentiated cell is a macrophage, a dendritic cell, or erythrocyte.
[8C] A pharmaceutical composition comprising the CMP or myeloid progenitor cell obtained by the method of any of [1C] to [5C], or the CMP-lineage differentiated cell obtained by the method of [6C] or [7C].
[9C] A method for treating or preventing a disease, comprising administering the CMP or myeloid progenitor cell obtained by the method of any of [1C] to [5C], the CMP-lineage differentiated cell obtained by the method of [6C] or [7C], or the pharmaceutical composition of [8C] to a patient in need thereof.
[10C] The CMP or myeloid progenitor cell obtained by the method of any of [1C] to [5C].
[11C] The CMP-lineage differentiated cell obtained by the method of [6C] or [7C].
[12C] A proliferation promoting agent of a CMP or a myeloid progenitor cell, comprising
   a molecule that suppresses expression of CDKN1A gene and/or p53 gene, or the function of an expression product thereof as an active ingredient, wherein
   the myeloid progenitor cell is a GMP, a macrophage progenitor cell, a dendritic cell progenitor cell, or an erythrocyte progenitor cell.
[13C] A CMP or a myeloid progenitor cell comprising a nucleic acid encoding an expression suppressive nucleic acid against CDKN1A gene operably linked to a fifth exogenous promoter, and/or a nucleic acid encoding an expression suppressive nucleic acid against p53 gene operably linked to a sixth exogenous promoter, wherein
   the myeloid progenitor cell is a GMP, a macrophage progenitor cell, a dendritic cell progenitor cell, or an erythrocyte progenitor cell.
[14C] A cell population comprising the CMP or myeloid progenitor cell of [13C] at a ratio of not less than 10% with respect to the whole cell population.
[15C] A cell preparation comprising the cell population of [14C] .

### [Advantageous Effects of Invention]

According to the present invention, the proliferative property of CMPs or myeloid progenitor cells can be improved by forcibly expressing a MYC family gene and BMI1 gene in any cell in the differentiation process from a hematopoietic progenitor cell into a specific myeloid progenitor cell.

In addition, the present invention enables stable proliferation of human iPS cell-derived hematopoietic progenitor cells for a long period of time in a doxycycline-inducible manner, suppresses proliferation by simply removing doxycycline from the medium at any timing, and can prepare at the same time large quantities of neutrophils, macrophages, erythroblasts, erythrocytes, and the like that have normal functions.

The present invention is capable of highly efficiently inducing immortalized myelocytic cell lines from various iPS cells. Therefore, by using iPS cells introduced with receptors targeting foreign body-specific antigens, genetically-modified iPS cells that increase cytotoxicity to target cells, HLA null iPS cells with suppressed immunorejection, and hereditary disease-iPS cells, the present invention is more physiological and can be expected to have superiority in both drug screening and cell therapy.

### [Brief Description of Drawings]

[Fig. 1]

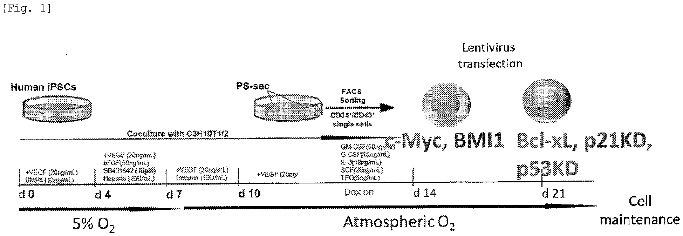

   Fig. 1 shows the method for establishing immortalized CMP cell line used in Example 1-1.
[Fig. 2]

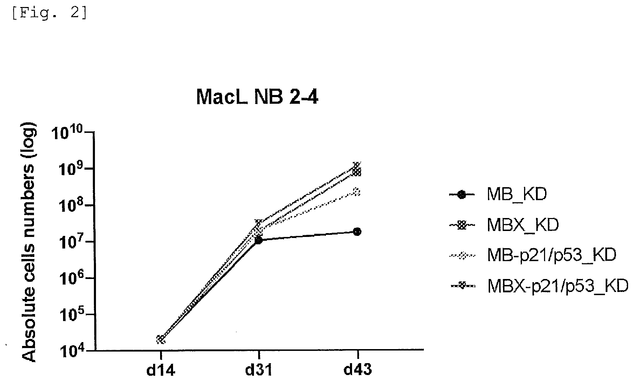

   Fig. 2 shows the results of cell proliferation numbers on days 14, 31, and 43 with respect to CMP cell line transfected with two genes: c-MYC/BMI1 after 14 days of culture and cultured as was (MB), MB cell line further transfected with BCL-XL gene using doxycycline-induced lentiviral vector after 21 days of culture (MBX), MB cell line further infected with persistently expressing sh p21/p53 lentiviral vector after 21 days of culture (MB-p21/p53_KD), and MBX cell line further infected with persistently expressing sh p21/p53 lentiviral vector after 21 days of culture (MBX-p21/p53_KD).
[Fig. 3]

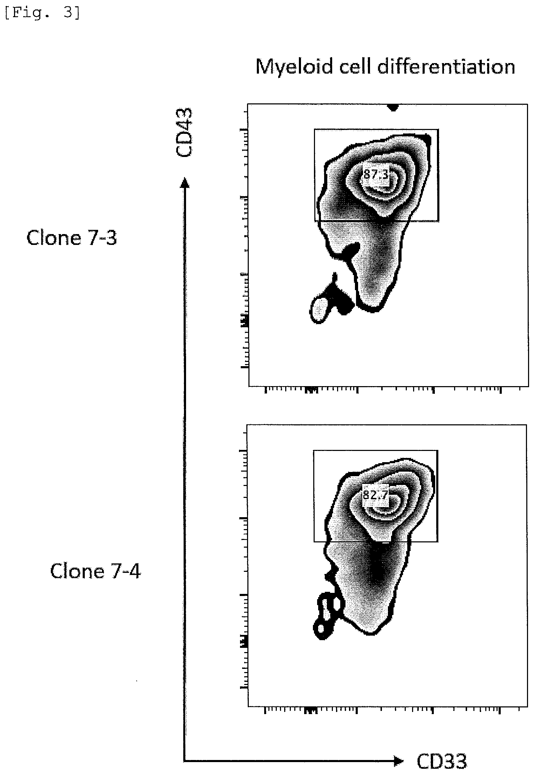

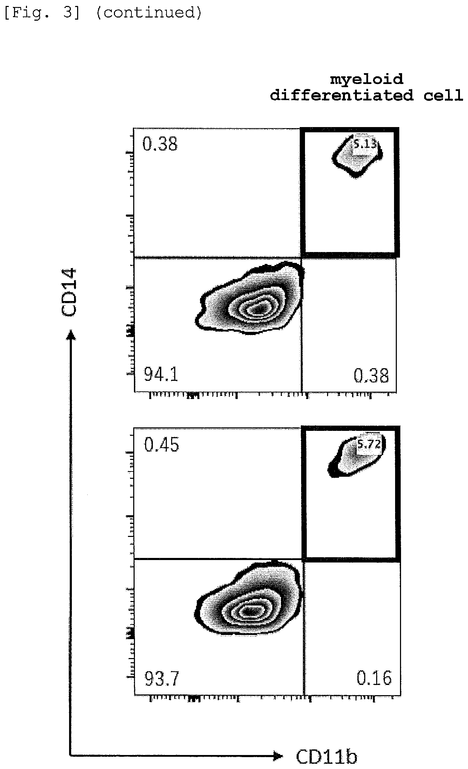

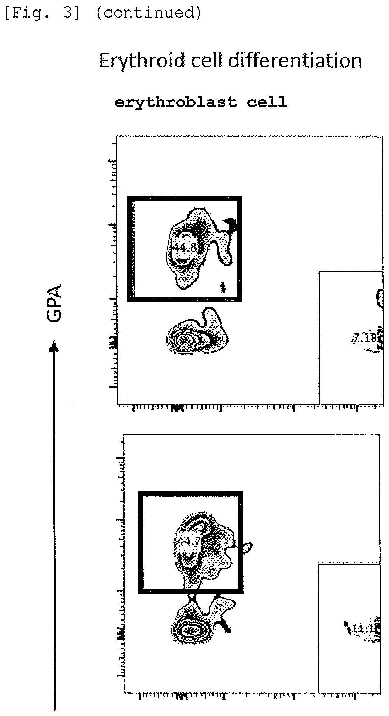

   Fig. 3 shows confirmation results of the CMP cell lines derived from healthy subject-derived iPS cells (upper row: Clone 7-3, lower row: Clone 7-4), with respect to which doxycycline was removed from the culture medium to suppress expression of three factors: MYC/BMI1/BCL-XL, and terminal differentiation into three major myelocytic lineages was confirmed on day 7.
[Fig. 4]

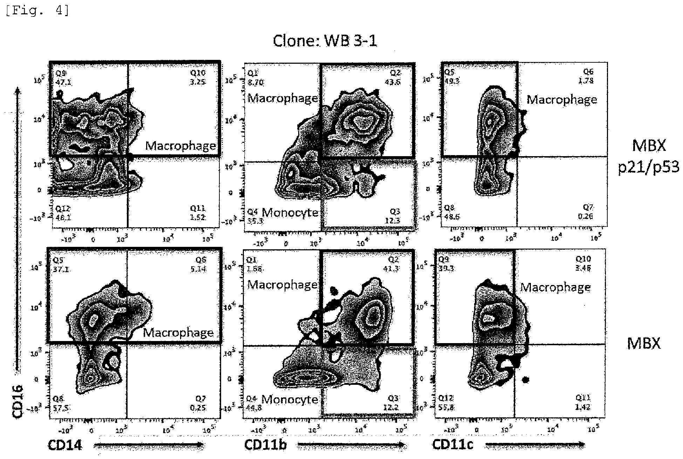

   Fig. 4 shows confirmation results of the MBX cell line and MBX-p21/p53_KD cell line, with respect to which doxycycline was removed from the culture medium to suppress expression of three factors: MYC/BMI1/BCL-XL, and differentiation into macrophage was confirmed on day 7.
[Fig. 5]

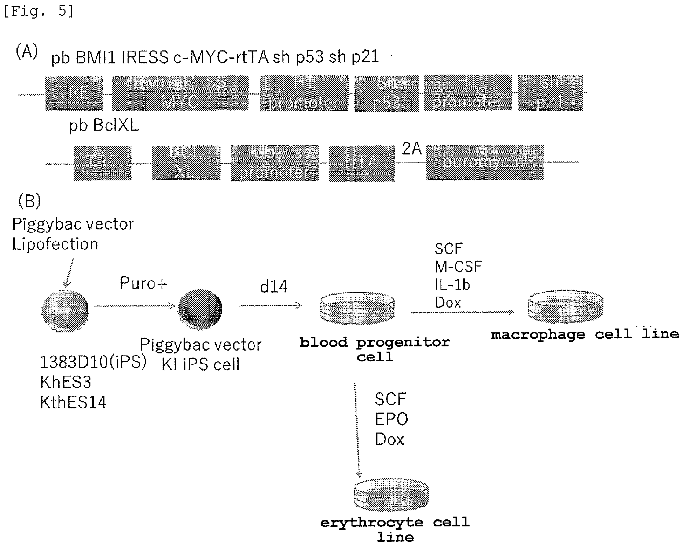

   Fig. 5 shows PiggyBac System used in Example 2 -1.
[Fig. 6]

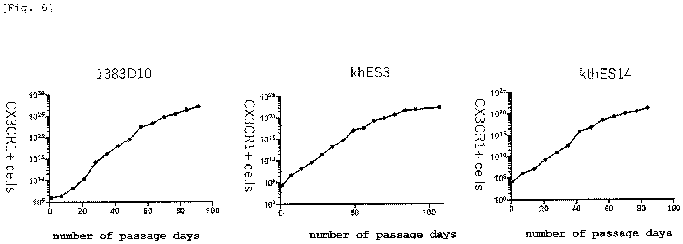

   Fig. 6 shows a proliferation curve of the macrophage cell line established in Example 2-1.
[Fig. 7]

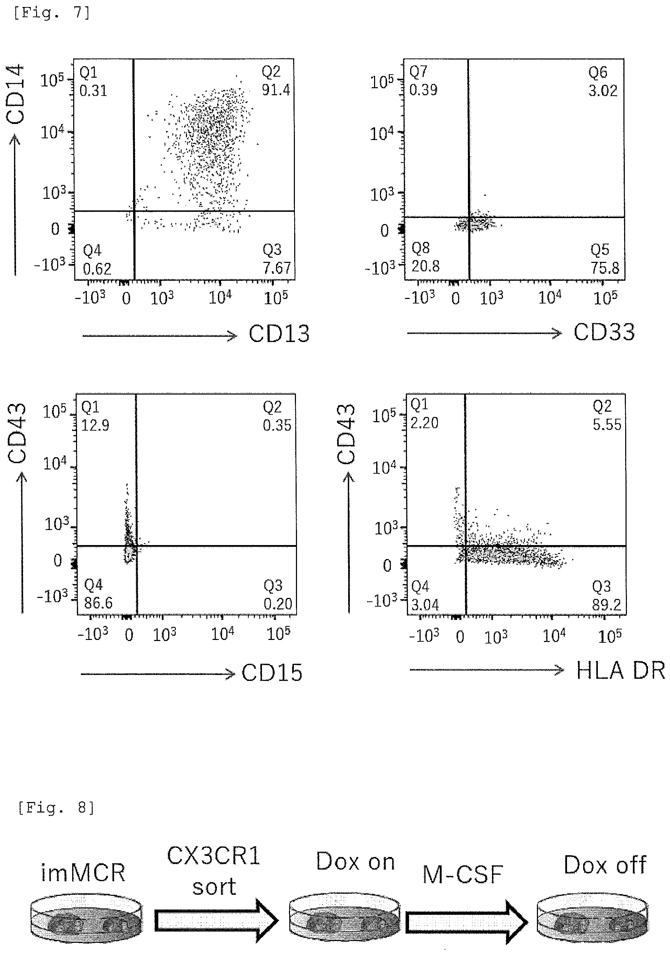

   Fig. 7 shows FACS analysis results of the 1383D10-derived macrophage cell line obtained in Example 2-1 which was stained with CD13, CD14, CD33, CD43, and HLA-DR.
[Fig. 8]

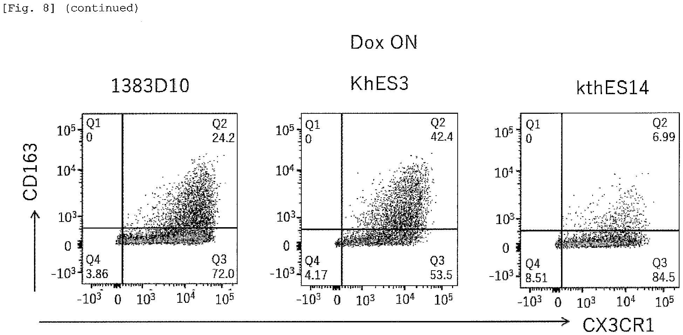

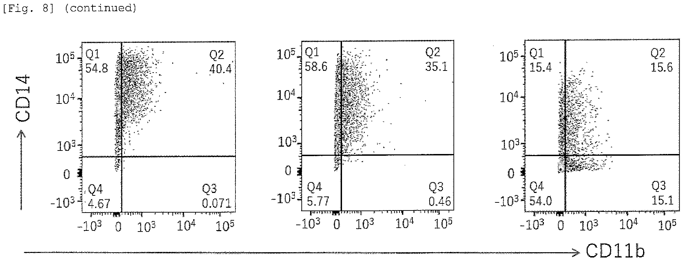

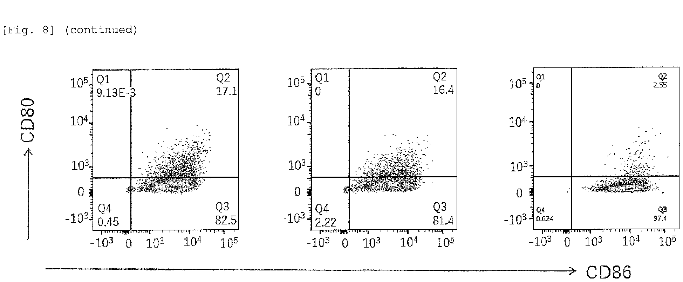

   Fig. 8, upper part, shows the operation of sorting each macrophage cell line obtained in Example 2-1 with a marker of macrophages: CX3CR1, and then leading them to gene expression (Dox on) and gene expression suppression (Dox off). Fig. 8, lower part, shows FACS analysis results of macrophage cell surface marker during gene expression (Dox on).
[Fig. 9]

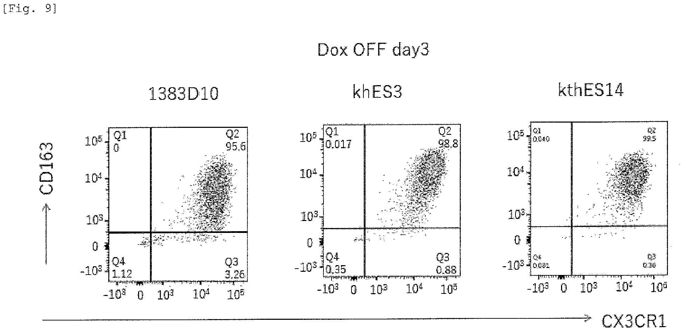

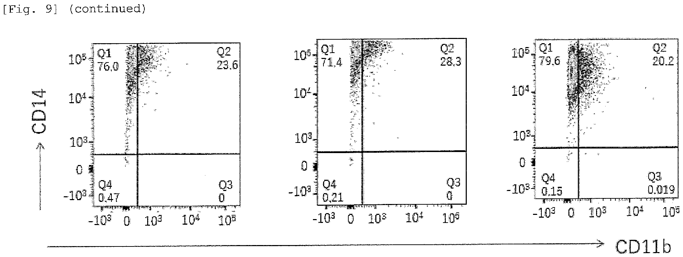

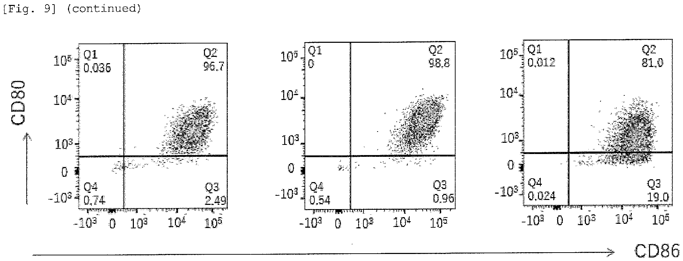

   Fig. 9 shows FACS analysis results of macrophage cell surface markers during gene expression suppression (Dox off) of each macrophage cell line obtained in Example 2-1.
[Fig. 10]

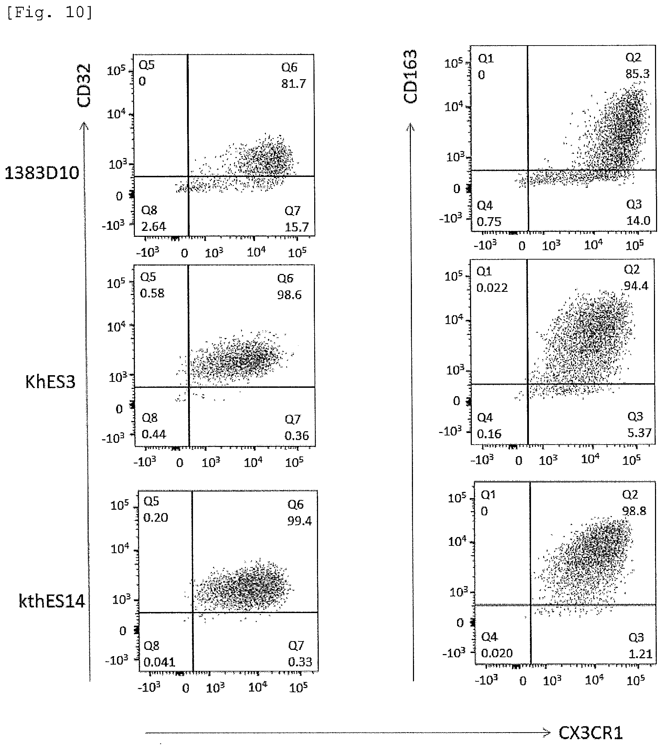

   Fig. 10 shows the results of verifying whether each macrophage cell line obtained in Example 2-1 is M1 type or M2 type.
[Fig. 11]

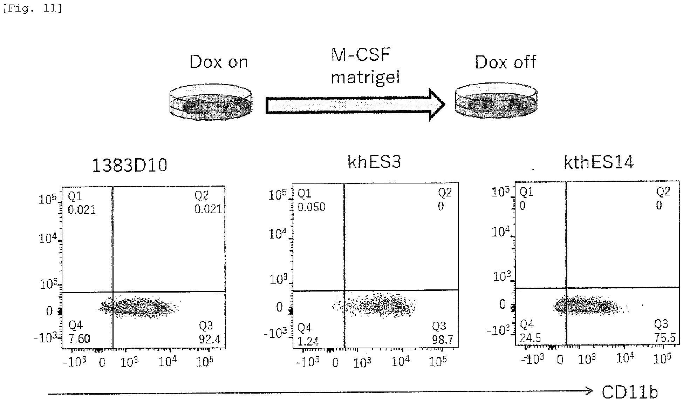

   Fig. 11 shows the results of removing doxycycline from the culture medium of each macrophage cell line obtained in Example 2-1 and staining with CD11b the cell line cultured on Matrigel with Dox off.
[Fig. 12]

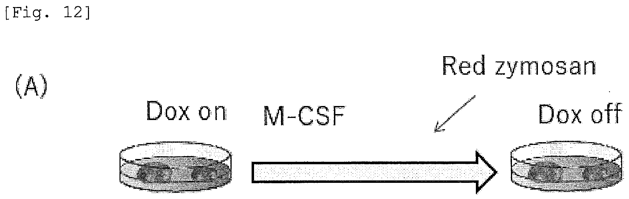

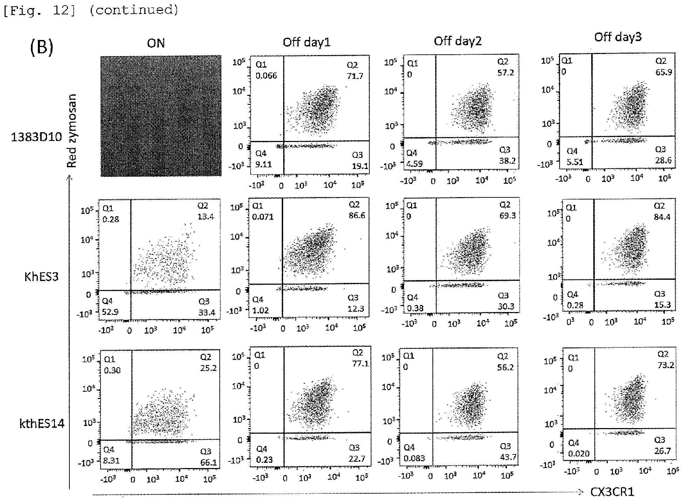

   Fig. 12 shows the results of examining the phagocytic ability of each macrophage cell line obtained in Example 2-1.
[Fig. 13]

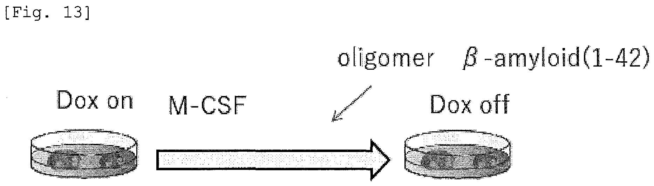

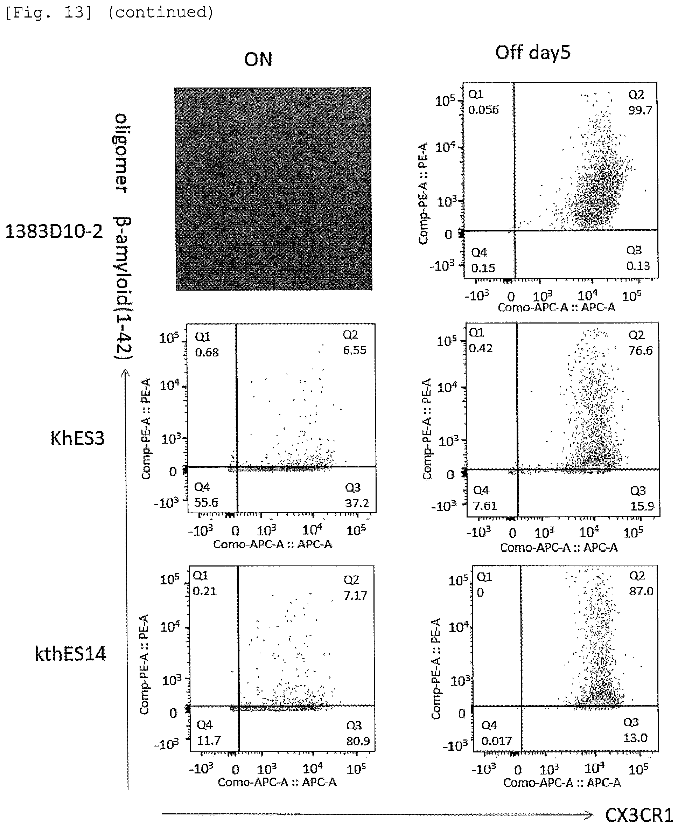

   Fig. 13 shows the results of examining the phagocytic ability of β-amiloid of each macrophage cell line obtained in Example 2-1.
[Fig. 14]

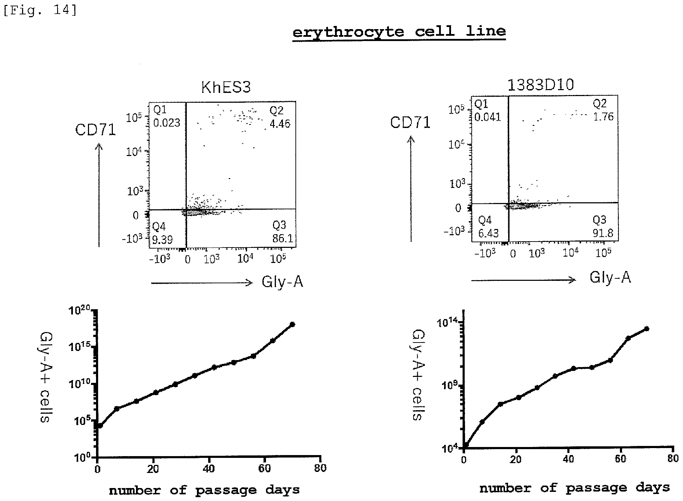

   Fig. 14 shows the results of counting the number of cells with glycophorin A (Gly-A) positive cells for the erythrocyte cell line established in Example 2-1.
[Fig. 15]

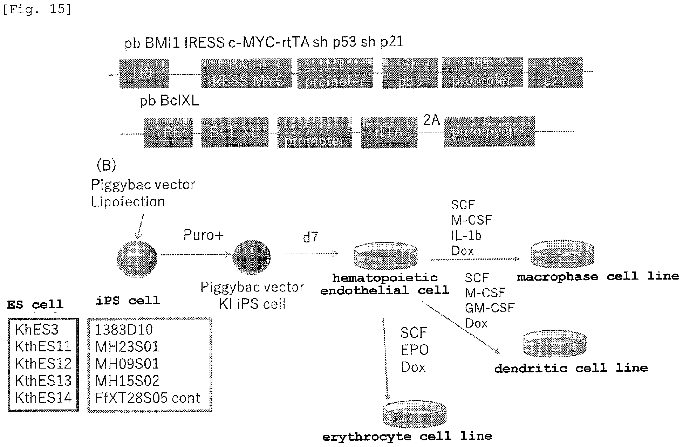

   Fig. 15 shows the PiggyBac System used in Example 3-1.
[Fig. 16]

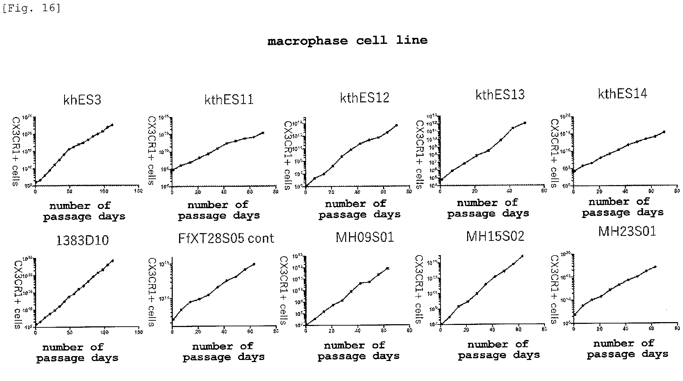

   Fig. 16 shows the proliferation curve of the macrophage cell line established in Example 3-1.
[Fig. 17]

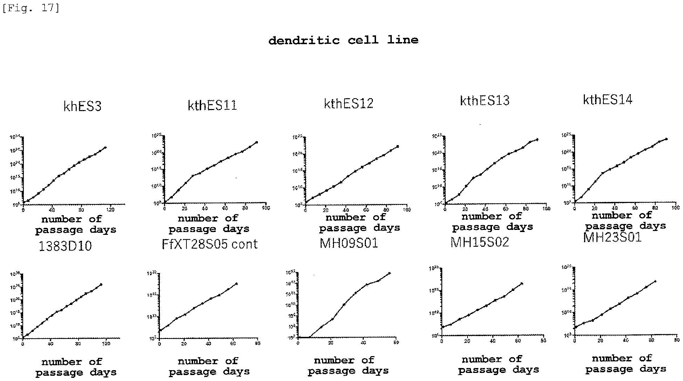

   Fig. 17 shows the proliferation curve of the dendritic cell line established in Example 3-1.
[Fig. 18]

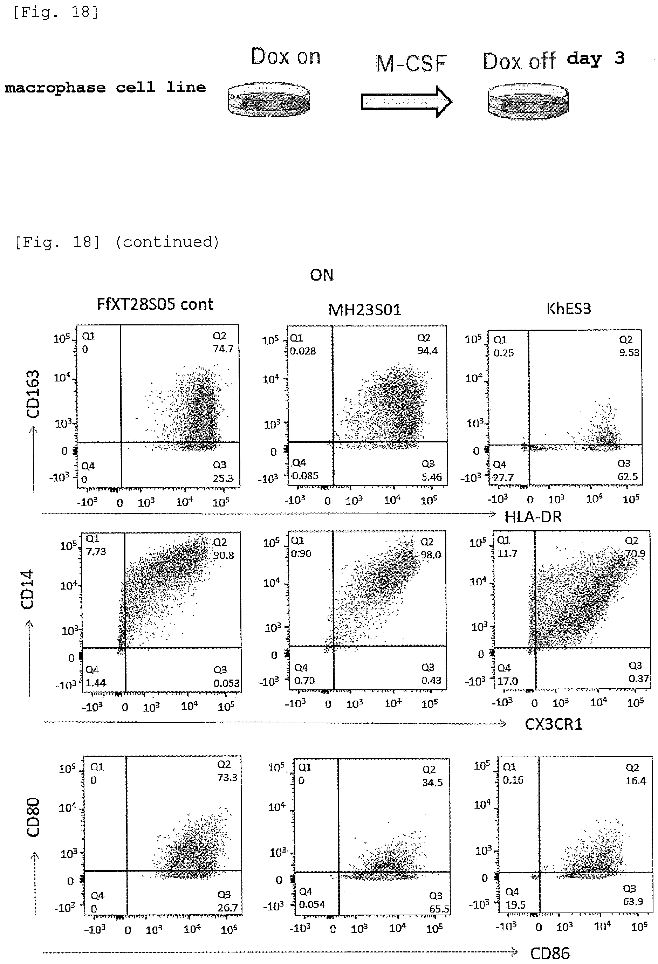

   Fig. 18, upper part, shows the operation of sorting each macrophage cell line obtained in Example 3-1 with a marker of macrophages: CX3CR1, and then leading them to gene expression (Dox on) and gene expression suppression (Dox off). Fig. 18, lower part, shows FACS analysis results of macrophage cell surface marker during gene expression (Dox on).
[Fig. 19]

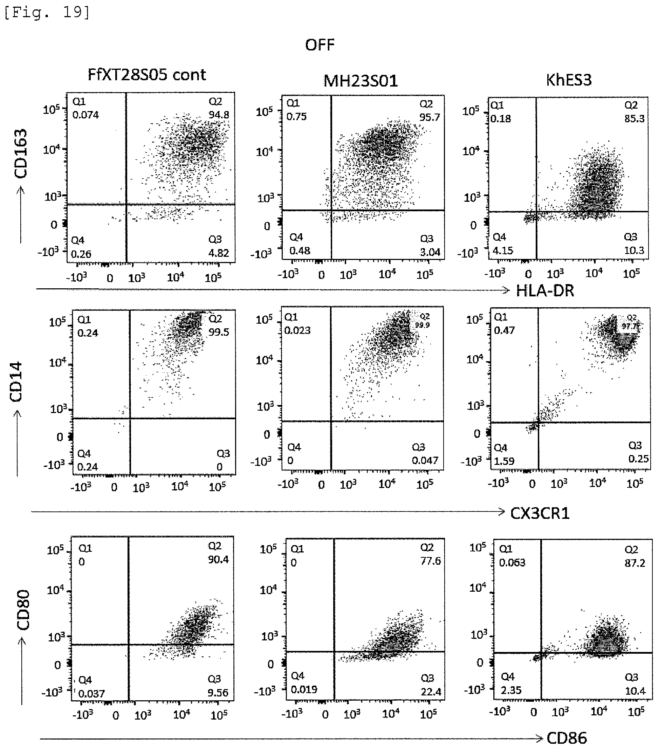

   Fig. 19 shows FACS analysis results of macrophage cell surface marker during gene expression suppression (Dox off), for each macrophage cell line obtained in Example 3-1.
[Fig. 20]

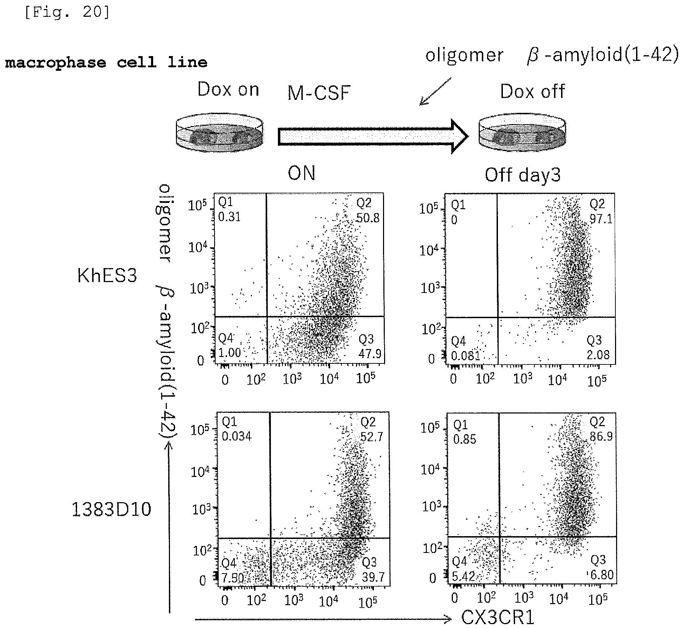

   Fig. 20 shows the results of examining the phagocytic ability of β-amiloid of each macrophage cell line obtained in Example 3-1.
[Fig. 21]

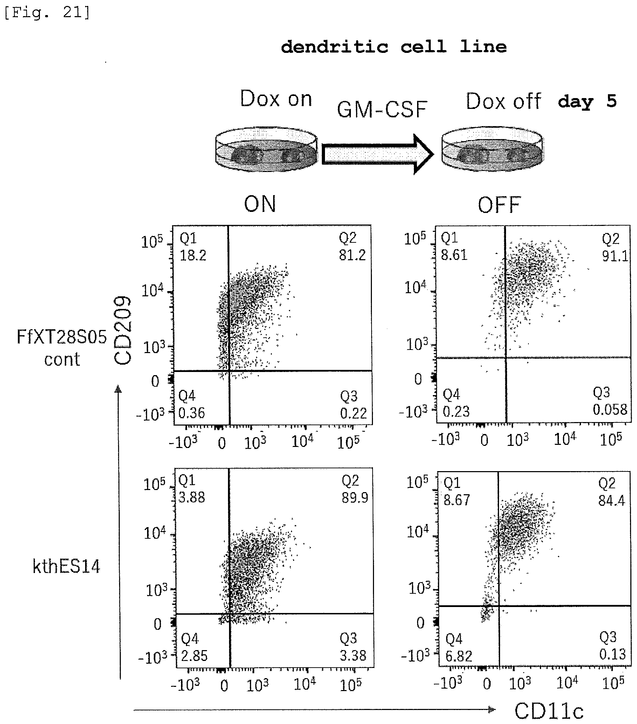

   Fig. 21, upper part, shows the operation of sorting each dendritic cell line obtained in Example 3-1 with a marker of dendritic cells: CD209, and then leading them to gene expression (Dox on) and gene expression suppression (Dox off). Fig. 21, lower part, shows FACS analysis results of dendritic cell surface marker during gene expression (Dox on) and gene expression suppression (Dox off).
[Fig. 22]

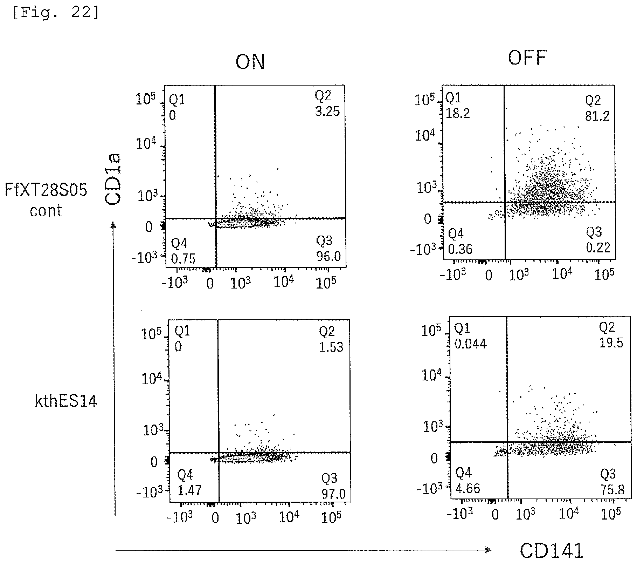

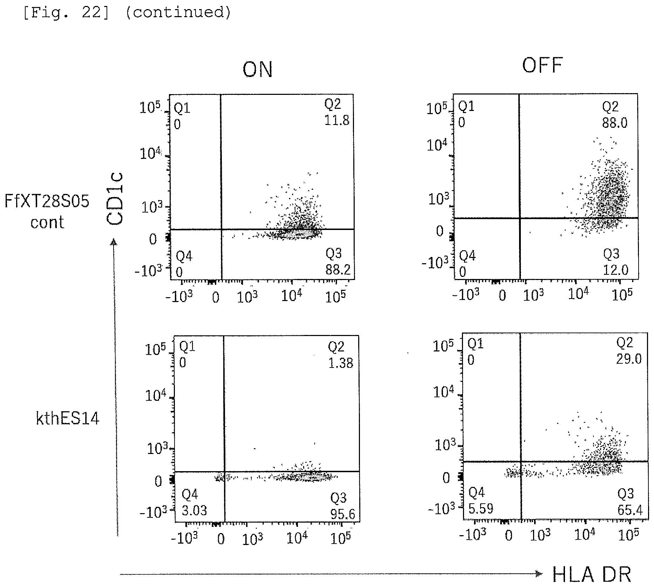

   Fig. 22 shows FACS analysis results of dendritic cell surface marker during gene expression (Dox on) and gene expression suppression (Dox off) for each dendritic cell line obtained in Example 3-1.
[Fig. 23]

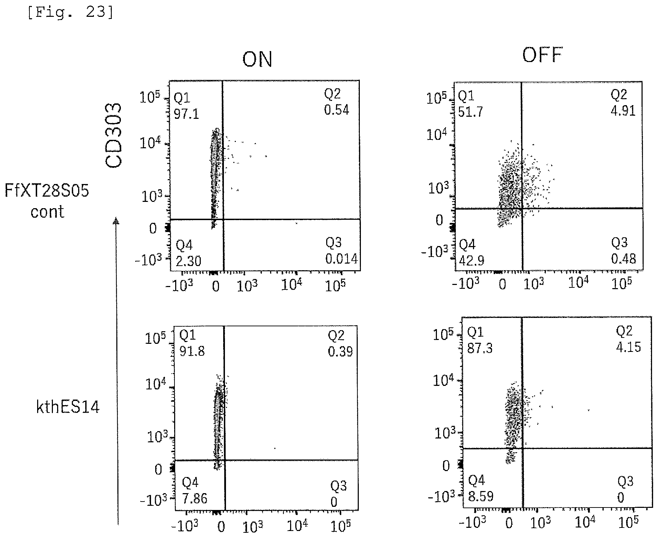

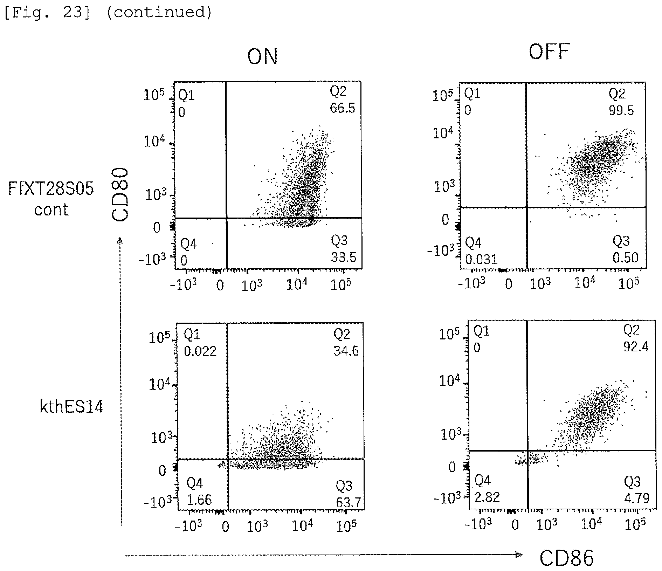

   Fig. 23 shows FACS analysis results of dendritic cell surface marker during gene expression (Dox on) and gene expression suppression (Dox off) for each dendritic cell line obtained in Example 3-1.
[Fig. 24]

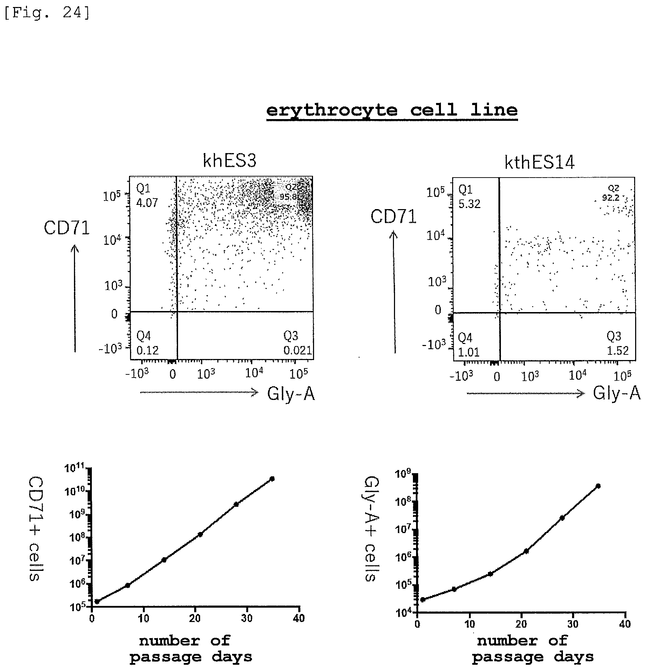

   Fig. 24 shows the results of counting the number of cells with Gly-A positive cells for the erythrocyte line established in Example 3-1.

### [Description of Embodiments]

### (1) Methodology 1

The method for improving proliferative property of CMPs or myeloid progenitor cells according to this embodiment includes a step of forcibly expressing a MYC family gene and BMI1 gene in any cell in the process of differentiation from hematopoietic progenitor cells into myeloid progenitor cells. As a result, it is expected that the proliferative property of CMPs or myeloid progenitor cells is improved, and an immortalized cell line that proliferates infinitely can be obtained.

The "process of differentiation from hematopoietic progenitor cells into myeloid progenitor cells" does not include hematopoietic progenitor cells. As the cell in the process of differentiation from hematopoietic progenitor cells into myeloid progenitor cells, CMP and myeloid progenitor cell to be described in detail below can be mentioned.

When used in the present specification, the "common myeloid progenitor" (CMP) is a progenitor cell that has the ability to differentiate into a monocyte-lineage cell such as macrophage and dendritic cell, a granulocyte-lineage cells such as neutrophil and basophil, platelet-producing megakaryocytes, and erythroid cells such as erythroblast cells and erythrocytes, but does not have the ability to differentiate into lymphocytes such as T cell, B cell and NK cell.

CMP can be characterized, for example, by the expression of cell surface marker of CD33⁺ in flow cytometry analysis. CMP can be obtained by culturing hematopoietic progenitor cells and hematopoietic endothelial cells under conditions suitable for inducing CMP differentiation. CMP can be obtained, for example, by culturing hematopoietic progenitor cells or hematopoietic endothelial cells in an appropriate medium containing GM-CSF, G-CSF, IL-3, SCF, and TPO for a period sufficient for differentiation into CMP. For example, 5% CO₂, 36 to 38°C, preferably 37°C, can be used as the culture environment. The induction of CMP can be confirmed by subjecting cultured cells to flow cytometric analysis to detect the appearance of cells with a pattern of the aforementioned cell surface marker expression characteristic of CMP, or by subjecting them to a colony formation assay to confirm that they have the aforementioned differentiation ability characteristic of CMP. The culture period until induction of CMP depends on the type of starting cells (hematopoietic progenitor cells, hematopoietic endothelial cells, etc.), and the presence thereof can be confirmed about 1 to 20 days after the start of differentiation induction.

"Hematopoietic progenitor cells" are hematopoietic lineage cells characterized as CD34⁺CD43⁺ cells, and may be, for example, cells derived from pluripotent stem cells such as ES cell and iPS cell. Particularly, cells obtained from net-like structures (also called "ES-sac" or "iPS-sac") prepared from pluripotent stem cells such as ES cells and iPS cells (particularly cells immediately after separation from the net-like structures) are preferred. As used herein, the "net-like structure" prepared from ES cell or iPS cell is a three-dimensional sac-like structure (with space inside) derived from ES cell or iPS cell, which is formed by endothelial cell population, etc., and containing hematopoietic progenitor cells inside. For net-like structures, see, for example, WO2008/041370; WO2009/122747; Lordier et al., Blood, 112:3164-3174 2009; TAKAYAMA et al., BLOOD 2008, 111:5298-5306.

"Hematopoietic endothelial cell" refers to cells that express VE-cadherin and have the ability to form colonies of both blood cells and vascular endothelial cells from a single cell (bidifferentiation potential). Hematopoietic endothelial cells can be VE-cadherin positive, CD41 positive, CXCR4 positive cells. "Hematopoietic endothelial cell" may be, for example, a cell derived from pluripotent stem cells such as ES cell and iPS cell, and is induced in the process of inducing net-like structures from ES cells or iPS cells.

Suitable cell culture conditions for preparing net-like structures from human pluripotent stem cells such as human ES cells and human iPS cells vary depending on the pluripotent stem cells to be used. For example, IMDM added with a final concentration of 15% FBS can be used as the medium, or other serum-free medium with growth factors and supplements added as appropriate can be used. In addition, 0 to 100 ng/ml, preferably about 20 ng/ml, of VEGF is preferably added for efficient formation of net-like structures. The culture environment varies depending on the type of ES cell or iPS cell to be used and, for example, conditions of 5% CO₂, 36 to 38°C, preferably 37°C, can be used. The culture period until formation of net-like structure varies depending on the type of pluripotent stem cell and induction conditions. In general, after seeding pluripotent stem cells on feeder cells, a cell aggregate containing hematopoietic endothelial cells is formed by about 7 days later, and a net-like structure containing hematopoietic progenitor cells is formed by about 14 to 16 days later.

The net-like structures formed are follicle-like structures, and concentrated hematopoietic progenitor cells are present inside. Hematopoietic endothelial cells contained in the cell aggregate and hematopoietic progenitor cells present inside the net-like structures can be separated by passing them through physical means, for example, sterilized sieve-like instruments (e.g., cell strainer, etc.).

"Myeloid progenitor cell" is a cell derived from CMP and differentiated from the CMP, and broadly refers to macrophage, dendritic cell, granulocyte, erythroblast, or progenitor cell of erythrocyte. CMP can differentiate into megakaryocyte-erythrocyte progenitor (MEP) and granulocyte-macrophage progenitor (GMP).

Thereafter, through multiple stages of differentiation, macrophages, dendritic cells, and granulocytes are produced from GMP, and megakaryocytes, erythroblasts, or erythrocytes are produced from MEP. That is, progenitor cell of macrophage, dendritic cell, granulocyte, erythroblast, or erythroid (myeloid progenitor cell) may be any cell in the process of differentiating from CMP into macrophage, dendritic cell, granulocyte, erythroblast, or erythroid, even though it is not the finally-differentiated macrophage, dendritic cell, granulocyte, megakaryocyte, erythroblast, or erythrocyte itself. Examples of the myeloid progenitor cell include, but are not limited to, MEP, GMP, macrophage progenitor cell, dendritic cell progenitor cell, erythrocyte progenitor cell, neutrophil progenitor cell, and the like. In the present invention, megakaryocyte progenitor cells (including those before multinucleation, described as "megakaryotic progenitor cell" in WO 2011/034073) in the process of differentiation from MEP into megakaryocytes are excluded from the myeloid progenitor cells.

MEP can be obtained by culturing hematopoietic progenitor cells, hematopoietic endothelial cells, or CMP under conditions suitable for inducing MEP differentiation. It can be obtained, for example, by culturing hematopoietic progenitor cells, hematopoietic endothelial cells, or CMP in an appropriate medium containing IL-3, SCF, and TPO for a period sufficient for differentiation into CMP. Macrophage progenitor cell can be obtained by culturing hematopoietic progenitor cells, hematopoietic endothelial cells, or CMP under conditions suitable for inducing macrophage progenitor cell differentiation. It can be obtained, for example, by culturing hematopoietic progenitor cells, hematopoietic endothelial cells, or CMP in an appropriate medium containing IL-1b, SCF, and M-CSF for a period sufficient for differentiation into macrophage progenitor cell. Dendritic cell progenitor cell can be obtained by culturing hematopoietic progenitor cells, hematopoietic endothelial cells, or CMP under conditions suitable for inducing dendritic cell progenitor cell differentiation. It can be obtained, for example, by culturing hematopoietic progenitor cells, hematopoietic endothelial cells, or CMP in an appropriate medium containing SCF, M-CSF, and GM-CSF for a period sufficient for differentiation into dendritic cell progenitor cell. Neutrophil progenitor cell can be obtained by culturing hematopoietic progenitor cells, hematopoietic endothelial cells, or CMP under conditions suitable for inducing neutrophil progenitor cell differentiation. It can be obtained, for example, by culturing hematopoietic progenitor cells, hematopoietic endothelial cells, or CMP in an appropriate medium containing SCF and GM-CSF for a period sufficient for differentiation into neutrophil progenitor cell. Erythrocyte progenitor cell can be obtained by culturing hematopoietic progenitor cells, hematopoietic endothelial cells, CMPs, or MEPs under conditions suitable for inducing erythrocyte progenitor cell differentiation. It can be obtained, for example, by culturing hematopoietic progenitor cells, hematopoietic endothelial cells, CMPs, or MEPs in an appropriate medium containing SCF and EPO for a period sufficient for differentiation into erythrocyte progenitor cell. For example, 5% CO₂, 36 to 38°C, preferably 37°C, can be used as the culture environment. The induction of each myeloid progenitor cell can be confirmed by subjecting cultured cells to flow cytometric analysis to detect the appearance of cells with a pattern of cell surface marker expression characteristic of each myeloid progenitor cell described below, or by subjecting them to a colony formation assay to confirm that they have the differentiation ability characteristic of each myeloid progenitor cell. The culture period until induction of CMP depends on the type of starting cells (hematopoietic progenitor cells, hematopoietic endothelial cells, etc.), and the presence thereof can be confirmed about 7 to 14 days after the start of differentiation induction.

CMP and MEP can be characterized, for example, by the following cell surface marker expression patterns in flow cytometry analysis.
CMP: Lin⁻/CD33⁺
MEP: CD41⁺ or CD41⁺Gly-A⁺

Other myeloid progenitor cells can be characterized, for example, by expression of at least one, preferably two or more, of the following cell surface markers in flow cytometric analysis.
macrophage progenitor cell: CX3CR1, CD16, CD14, CD11b, CD13, CD86
dendritic cell progenitor cell: CD209, CD11c, CD303, CD80, CD86
erythrocyte progenitor cell: Gly-A, CD71
neutrophil progenitor cell: CD15, CD16

The present inventors have reported that oncogenes such as MYC and genes such as BMI1 are forcibly expressed in megakaryocytes before multinucleation and derived from pluripotent stem cells (including those described as "megakaryocyte" in WO2011/034073), thereby increasing the proliferation ability of the megakaryocytes (WO2011/034073, JEM, 207:2817-2830 2010). The present invention is based on the finding that this methodology is applicable not only to megakaryocytes but also to any cells in the process of differentiation from hematopoietic progenitor cells into myeloid progenitor cells and can enhance the proliferative potency thereof.

When used in the present specification, the "gene expression" means that the DNA encoding the gene of interest is transcribed into mRNA and/or the mRNA is translated into protein. The forced expression of the MYC family gene and the BMI1 gene may be performed simultaneously or successively. After forced expression, cells may be passage cultured, and the period from the last passage to the date of removal of forced expression is not particularly limited, and may be, for example, one day, two days, or three days or more. When maintaining proliferation of CMPs or myeloid progenitor cells, it is preferable to maintain forced expression of MYC family gene and BMI1 gene during the culture period.

The MYC family gene is a gene that induces canceration of cells in vivo. Examples of the MYC family gene include c-MYC, N-MYC, and L-MYC genes. Among these, c-MYC is preferred.

The BMI gene is a gene that downregulates CDKN2a (INK4A/ARF) gene and functions to avoid cellular senescence (Ogura et al, Regenerative Medicine, vol. 6, No. 4, pp 26-32; Jseus et al., Jseus et al., Nature Reviews Molecular Cell Biology vol. 7, pp 667-677, 2006; Proc. Natl. Acad. Sci. USA, vol. 100, pp 211-216, 2003).

The method for improving proliferative property of CMPs or myeloid progenitor cells may further include a step of extracting (isolating or purifying) cells at a specific desired stage of differentiation (CMPs or myeloid progenitor cells). The cells at a specific stage of differentiation may be extracted before forcibly expressing the MYC family gene and the BMI1 gene (CMPs or myeloid progenitor cells). In one embodiment, this extraction step is performed before forcibly expressing the MYC family gene and the BMI1 gene, and the MYC family gene and the BMI1 gene are forcibly expressed in the extracted cells at a specific stage of differentiation (CMPs or myeloid progenitor cells). In another embodiment, a cell population containing cells at a specific desired stage of differentiation (CMPs or myeloid progenitor cells) in which the MYC family gene and the BMI1 gene have been forcibly expressed is prepared, and then the cells at a specific desired stage of differentiation (CMPs or myeloid progenitor cells) are extracted from the cell population. The MYC family gene and BMI1 gene may continuously be expressed forcibly in these extracted cells at a specific stage of differentiation (CMPs or myeloid progenitor cells). By extracting the cells of interest and applying the method of the present invention to the extracted cells, or by extracting the cells of interest from a cell population to which the method of the present invention has been applied and continuously culturing the cells, the cell type of interest can be efficiently proliferated. From the aspect of efficiently improving the proliferative property of the extracted cell type, the cell to be extracted is preferably only the cell line of CMP or only a single type of cell line of myeloid progenitor cell, or may be a cell population of a mixture of two or more types of these cells.

Examples of the cells to be extracted include CMP, MEP, GMP, macrophage progenitor cell, dendritic cell progenitor cell, erythrocyte progenitor cell, neutrophil progenitor cell, and the like. Extraction of the cells of interest can be performed by methods known to those skilled in the art, such as flow cytometry, panning, magnetic beads, and the like, using antibodies to cell surface markers that are specifically expressed (or not expressed) on the cell. Isolation of CMP and MEP can be performed by isolating cells that satisfy the cell surface marker expression pattern described above. When extracting macrophage progenitor cells, for example, cells positive for at least one cell surface marker selected from the group consisting of CX3CR1, CD16, CD14, CD11b, CD13, and CD86 (preferably CX3CR1) are isolated. When extracting dendritic cell progenitor cells, for example, cells positive for at least one cell surface marker selected from the group consisting of CD209, CD11c, CD303, CD80, and CD86 (preferably CD209) are isolated. When extracting erythrocyte progenitor cells, for example, cells positive for at least one cell surface marker selected from the group consisting of Gly-A and CD71 (preferably Gly-A) are isolated. When extracting neutrophil progenitor cells, for example, cells positive for at least one cell surface marker selected from the group consisting of CD15 and CD16 are isolated.

The cells of interest may be isolated such that the percentage of the cells of interest in the cell population after the extraction operation is, for example, not less than 10%, not less than 20%, not less than 30%, not less than 40%, not less than 50%, not less than 60%, not less than 70%, not less than 80%, not less than 90%, not less than 95% (e.g., 100%). Single cells of the cells of interest may be isolated.

The method for improving proliferative property of CMPs or myeloid progenitor cells according to the present invention may further include a step of forcibly expressing BCL-XL gene in the CMPs or myeloid progenitor cells. By expressing the BCL-XL gene in addition to the MYC family gene and BMI1 gene, further promotion of proliferation of CMP or myeloid progenitor cells can be expected. The period of the above-mentioned forced expression of BCL-XL gene can be appropriately determined by those of ordinary skill in the art.

The BCL-XL gene is a gene that functions to suppress cell apoptosis.

The forced expression of the MYC family gene, BMI1 gene, and/or BCL-XL gene may be performed simultaneously or successively. For example, the MYC family gene and the BMI1 gene may be forcibly expressed and the BCL-XL gene may successively be expressed forcibly to obtain CMPs or myeloid progenitor cells with improved proliferation potency. In addition, CMPs or myeloid progenitor cells with improved proliferation potency can also be obtained by forcibly expressing simultaneously the MYC family gene, the BMI1 gene, and the BCL-XL gene. When maintaining proliferation of CMPs or myeloid progenitor cells, it is preferable to maintain forced expression of the MYC family gene and BMI1 gene, as well as BCL-XL, during the culture period.

The MYC family gene, BMI1 gene, and BCL-XL gene promote proliferation of CMPs or myeloid progenitor cells, but can inhibit terminal differentiation of CMP-lineage differentiated cells (e.g., macrophage, dendritic cell, neutrophil, erythrocyte). Thus, expression of these genes may be suppressed before entering the terminal differentiation step. Suppressing the expression of these genes in CMPs or myeloid progenitor cells facilitates the induction of functional, more mature CMP-lineage differentiated cells (e.g., macrophage, dendritic cell, neutrophil, erythrocyte).

When a gene such as the MYC family gene, BMI1 gene and BCL-XL gene is forcibly expressed in a cell, any method well known to those of ordinary skill in the art may be employed. For example, the gene may be introduced and expressed in cells using a gene delivery system with a viral vector such as lentivirus and retrovirus, or non-viral vector such as plasmid vector and episomal vector. It is also preferable to use a method of non-virulently incorporating the target gene into the genome of cell by using transposon, establishing a stable expression cell line, and then removing unwanted transgene by transposonase (e.g., PiggyBac Transposon system). CMPs or myeloid progenitor cells may be transfected with an expression vector (e.g., viral vector) of the desired gene (e.g., MYC family gene and BMI1 gene, optionally further BCL-XL gene), or CMPs or myeloid progenitor cells may be induced from pluripotent stem cells (e.g., ES cells, iPS cells), hematopoietic progenitor cells, or hematopoietic endothelial cells that have been incorporated in advance with expression cassettes for the desired gene (e.g., MYC family gene and BMI1 gene, optionally further BCL-XL gene), and the gene may be forcibly expressed at this stage. Alternatively, differentiation of pluripotent stem cells, hematopoietic progenitor cells, or hematopoietic endothelial cells into CMPs or myeloid progenitor cells may be induced while forcibly expressing said gene in the pluripotent stem cells (e.g., ES cells, iPS cells), hematopoietic progenitor cells, or hematopoietic endothelial cells that have been incorporated in advance with expression cassettes for the desired gene (e.g., MYC family gene and BMI1 gene, optionally further BCL-XL gene). When gene expression is performed by a gene delivery vector, the gene can be operably linked downstream of an appropriate promoter, inserted into the gene delivery vector, and then introduced into the cell to express the target gene. The promoter can be an exogenous promoter. In the present specification, an "endogenous" promoter of a gene means a promoter that is naturally linked to the gene in the genome, and an "exogenous" promoter of a gene means one that is artificially placed proximal to the gene by genetic manipulation (i.e., molecular biological techniques) such that transcription of the gene is directed by an operably linked promoter. As used herein, linking "operably" means linking the promoter to the target gene so that the target gene is controlled in cis by the promoter and the desired expression of the target gene is achieved. The exogenous promoter may be a constitutive promoter or a regulatable promoter. Examples of the constitutive promoter include CMV promoter, EF1 promoter, ubiquitin promoter, and the like. The regulatable promoter means an inducible or derepressible promoter and refers to a promoter that can bind to either a repressor or an inducer and has a DNA sequence that works with the promoter. When the promoter is induced or derepressed, it becomes "on" and when the promoter is not induced or derepressed, it becomes "off". Examples of the regulatable promoter include drug-responsive promoters such as tetracycline-responsive promoter, steroid-responsive promoter, metallothionein promoter, and the like. Tetracycline-responsive promoters are known regulatable promoters that are reversibly controlled by the presence or absence of tetracycline or a derivative thereof (e.g., doxycycline (Dox)). Tetracycline-responsive promoters are promoters with internal tetracycline response elements (TREs) and are promoters that are activated (i.e., induce expression of the target protein) by binding of reverse tetracycline-regulated trans-activator (rtTA) protein or tetracycline-regulated trans-activator (tTA) to the TRE. The rtTA protein binds to the TRE in the presence of Dox, while the tTA protein binds to the TRE in the absence of Dox to induce expression of the target gene functionally linked to the promoter downstream of the TRE sequence. When a tetracycline-responsive promoter is used, by culturing cells transfected with the gene functionally linked to a tetracycline-responsive promoter and rtTA or tTA protein in the presence of Dox, the expression of the gene can be induced or suppressed in a Dox-dependent manner. The exogenous promoter is preferably a regulatable promoter. By using a regulatable promoter, the target gene can also be inducibly expressed by controlling, for example, the addition of drugs. Such gene expression system with drugs can be easily selected by those skilled in the art to achieve the desired regulation of expression of the MYC family gene, the BMI1 gene, the BCL-XL gene, and the like. Commercially available kits and the like may be used to perform such expression. The MYC family gene, BMI1 gene, and BCL-XL gene, which are the target genes for controlling expression, may be inserted in separate vectors or in the same vector.

Suppression of the expression of the MYC family gene, BMI1 gene, BCL-XL gene, and the like in cells may be achieved, for example, by removing the drug and the like to release the induction of expression by the drug-induced expression system using the aforementioned regulatable promoter. Alternatively, the transfected MYC family gene, BMI1 gene, BCL-XL gene, and the like can be removed using a Cre/lox system or the like to suppressively control the expression of these genes. Commercially available kits and the like may also be used as appropriate for suppressive control of the expression of the MYC family gene, BMI1 gene, BCL-XL gene, and the like.

Commercially available drug-responsive gene expression induction systems such as Tet-on (registered trademark) or Tet-off (registered trademark) system may be used for forced expression and release of the forced expression of each of the above-mentioned genes. In this case, in the step of forced expression, the corresponding drug, for example, tetracycline or doxycycline, may be included in the medium, and the forced expression may be suppressed by removing same from the medium.

Forced expression and release of the forced expression of genes can be performed by the methods described in WO 2011/034073 (supra) and US 2012/0238023, WO 2012/157586 (supra), US 2014/0127815, WO 2014/123242, US 2016/0002599, and Nakamura S et al, Cell Stem Cell. 14, 535-548, 2014, or other known methods or methods analogous thereto.

The method for improving proliferative property of CMPs or myeloid progenitor cells of the present invention may include a step of suppressing the expression of at least one of CDKN1A gene and p53 gene, or the function of expression products thereof, in the CMPs or myeloid progenitor cells. As used herein, the expression is used under a concept including transcription and translation, and inhibition of expression can include inhibition at the level of transcription as well as inhibition at the level of translation. In the method of the present invention, suppressing the expression of the CDKN1A gene and/or p53 gene, or the function of expression products thereof, can be expected to further improve the proliferation of CMPs or myeloid progenitor cells.

CDKN1A (cyclin-dependent kinase inhibitor 1A) gene encodes the cell cycle inhibitor p21, and is also known as a downstream gene of the p53 gene, which is a tumor suppressor gene. Activated p53 protein acts as a transcription factor and increases the expression of the p53 downstream gene group. When used in the present specification, "suppressing the expression of gene or the function of expression products thereof" may be achieved by directly suppressing the expression of the target gene or the function of expression products thereof (e.g., p21 in the case of CDKN1A gene), or may be achieved by regulating the expression of genes upstream of the target gene or the function of expression products thereof. However, in the present specification, when expression of CDKN1A gene, or the function of expression products thereof is suppressed, genes upstream of the target CDKN1A gene do not include p53 gene, and further, INK4A gene and ARF gene, which are other cancer suppressor genes upstream of the p53 gene.

It is preferable to suppress the expression of not only the CDKN1A gene but the p53 gene, or the function of expression products thereof.

Suppression of the expression of the above-mentioned respective genes or the function of expression products thereof can be performed by a known method. For example, it can be performed by introducing various molecules into cells, such as siRNA, shRNA, and antisense nucleic acid ("expression suppressor nucleic acid") that can specifically suppress the expression of each gene, expression vectors that can express these expression suppressor nucleic acids, and the like. Alternatively, other technologies, such as genome editing, may be used to knock down the gene. For example, when a gene is knocked down using the CRISPR-Cas system, a guide RNA targeting the gene and a fusion protein of an inactivated Cas and a repressor domain, such as dCas, and the like are used.

siRNA is typically a double-stranded oligoRNA consisting of RNA having a sequence complementary to the nucleotide sequence of the mRNA of the target gene or a partial sequence thereof and its complementary strand. The length of siRNA is generally about 19 to 30 bases, preferably 21 to 25 bases, when used in mammalian cells. The nucleotide sequences of these RNAs can be designed by the skilled person according to the sequence information of the gene whose expression is to be suppressed. shRNAs can also be used instead of siRNAs.

Antisense nucleic acid means a nucleic acid that contains a nucleotide sequence that can hybridize specifically with the target mRNA under physiological conditions of a cell expressing the target mRNA (mature mRNA or early transcript) and can inhibit the translation of the polypeptide encoded by the target mRNA in the hybridized state. Antisense nucleic acids are generally single-stranded nucleic acids of 10 to 100 bases in length, preferably 15 to 30 bases in length. The type of antisense nucleic acid may be DNA or RNA, or a chimera of DNA and RNA. The nucleotide sequences of antisense nucleic acids can be designed by the skilled person according to the sequence information of the gene whose expression is to be suppressed.

In addition to the above-mentioned techniques, compounds known to inhibit the expression of each gene can also be used. For example, p21 inhibitors such as UC2288, butyrolactone I, LLW10, sorafenib, and sterigmatocystin are known as compounds that suppress CDKN1A gene expression. In addition, Pifithrin α, Nutlin-3, ReACp53, and RG7388, and the like are known as p53 inhibitors.

Alternatively, in order to suppress the expression of the gene or the function of expression products thereof, the target gene may be knocked out using known techniques. Knockout of a gene means that all or a part of the gene has been destroyed or mutated so that it does not perform its original function. The gene may be destroyed or mutated so that one allele on the genome does not function. Multiple alleles may also be destroyed or mutated. Knockout can be performed by known methods. For example, a method of knocking out a target gene by introducing a DNA construct into a cell that has been engineered to cause genetic recombination with the target gene, or a method of knocking out by introducing insertion, deletion, or substitution of bases using genome editing techniques such as TALEN and CRISPR-Cas system can be mentioned.

In addition, compounds that suppress the transcription and transcription products of each gene, or inhibitors of the binding of the produced protein to the target protein (p53 binding inhibition: Pifithrin α, Nutlin-3, ReACp53, RG7388, and the like; p21 binding inhibition: UC2288, butyrolactone I, LLW10, sorafenib, sterigmatocystin, etc.) and the like may also be used.

Suppression of gene expression or the function of expression products thereof can be performed by the aforementioned methods.

Suppression of the expression of the CDKN1A gene and/or the p53 gene is preferably performed by introducing, into the cells, expression vectors expressing the expression suppressor nucleic acids for the respective genes. When an expression suppressor nucleic acid for a gene such as CDKN1A gene, p53 gene, or the like is forcibly expressed in a cell, any method known to the artisan may be used. For example, a nucleic acid encoding the expression suppressor nucleic acid may be introduced and expressed in cells using a gene delivery system with a viral vector such as lentivirus, retrovirus, or the like or non-viral vector such as plasmid vector, episomal vector, or the like. It is also preferable to use a method of non-virulently incorporating a nucleic acid encoding expression suppressor nucleic acid into the genome of cell by using transposon, establishing a stable expression cell line of the expression suppressor nucleic acid, and then removing unwanted introduced nucleic acid by transposonase (e.g., PiggyBac Transposon system). CMPs or myeloid progenitor cells may be transfected with an expression vector (e.g., viral vector) of an expression suppressor nucleic acid for the desired gene (e.g., CDKN1A gene, p53 gene), or CMPs or myeloid progenitor cells may be induced from pluripotent stem cells (e.g., ES cells, iPS cells), hematopoietic progenitor cells, or hematopoietic endothelial cells that have been incorporated in advance with expression cassettes of an expression suppressor nucleic acid for the desired gene (e.g., CDKN1A gene, p53 gene), and the siRNA, shRNA, or antisense nucleic acid may be forcibly expressed at this stage. Alternatively, differentiation of pluripotent stem cell, hematopoietic progenitor cell, or hematopoietic endothelial cell into CMPs or myeloid progenitor cells may be induced while forcibly expressing the expression suppressor nucleic acid in pluripotent stem cells (e.g., ES cells, iPS cells), hematopoietic progenitor cells, or hematopoietic endothelial cells, in each of which an expression cassette of an expression suppressor nucleic acid for the desired gene (e.g., CDKN1A gene, p53 gene) has been incorporated in advance. When the expression suppressor nucleic acid is expressed using an expression vector, a nucleic acid encoding (e.g., DNA) the expression suppressor nucleic acid can be linked downstream of an appropriate promoter, inserted into the expression vector, and then introduced into the cell to express the expression suppressor nucleic acid of interest. The promoter can be an exogenous promoter. The exogenous promoter may be a constitutive promoter or a regulatable promoter, preferably a constitutive promoter. As an example of the constitutive promoter, when comparatively small RNA such as siRNA, shRNA, or the like is expressed, a pol III promoter such as U6 promoter, H1 promoter, tRNA promoter, retroviral LTR promoter, adenovirus VAI promoter, 5S rRNA promoter, 7SK RNA promoter, or 7SL RNA promoter is preferably used. The nucleic acid encoding the expression suppressor nucleic acid for the CDKN1A gene and the nucleic acid encoding the expression suppressor nucleic acid for the p53 gene may be inserted into separate expression vectors, or may be inserted into the same expression vector.

In this aspect, the suppression of the expression of the CDKN1A gene or p53 gene or the function of expression products thereof may be performed simultaneously with the forced expression of any of the MYC family gene, BMI1 gene, and BCL-XL gene. It is preferably performed simultaneously with the forced expression of BCL-XL gene or thereafter. For example, it can be performed after confirmation of a decrease in the cell proliferation. In one embodiment, the cell proliferation rate at a certain time point is compared to the most recent cell proliferation rate (for example, when cell proliferation is confirmed every week, the cell proliferation rate of a certain week is compared with the proliferation rate of one week before), and it can be performed after confirmation of the state in which the proliferation rate has decreased to 1/2 or less. Although not intended to be limiting, a decrease in cell proliferation can be seen immediately after forced expression of MYC family gene and BMI1 gene until about 30 days later, 40 days later, 50 days later, 60 days later, 70 days later, 80 days later, or 90 days later. In one embodiment, in CMPs or myeloid progenitor cells, the MYC family gene (e.g., c-Myc gene) and the BMI1 gene are forcibly expressed and, in parallel, the expression of CDKN1A gene and/or p53 gene or the function of expression products thereof is suppressed. In one embodiment, in CMPs or myeloid progenitor cells, the MYC family gene (e.g., c-Myc gene), the BMI1 gene, and the BCL-XL gene are forcibly expressed and, in parallel, the expression of the CDKN1A gene and/or p53 gene or the function of expression products thereof is suppressed.

Each of the MYC family genes, BMI1 gene, BCL-XL gene, CDKN1A gene, p53 gene, and the like as used herein means those genes encoded by their known nucleic acid sequences, for example, cDNA sequences. Each gene can also include homologs that are identified based on the homology of known nucleic acid sequences. A "homolog" is a gene whose cDNA sequence is substantially identical to the nucleic acid sequence of the gene.

Among the MYC family genes, a homologue of the c-MYC gene is a gene whose cDNA sequence is substantially the same as, for example, the nucleic acid sequence shown in SEQ ID NO: 1. A cDNA consisting of a sequence substantially the same as the nucleic acid sequence shown in SEQ ID NO: 1 refers to a DNA consisting of a sequence having not less than about 60%, preferably not less than about 70%, more preferably not less than about 80%, for example, 81%, 82%, 83%, 84%, 85%, 86%, 87%, 88%, 89%, further preferably about not less than 90%, for example, 91%, 92%, 93%, 94%, 95%, 96%, 97%, 98%, most preferably not less than about 99%, identity with the DNA consisting of the sequence represented by SEQ ID NO: 1, or a DNA that can hybridize under stringent conditions with DNA or RNA consisting of a sequence complementary to the nucleic acid sequence shown in SEQ ID NO: 1, wherein proteins encoded by these DNAs inhibit the cell cycle. Alternatively, the cDNA consisting of a sequence substantially the same as the nucleic acid sequence shown in SEQ ID NO: 1 refers to a DNA consisting of a sequence shown in SEQ ID NO: 1 in which one or more, for example, 1 to 10, preferably several, for example, 1 to 5, 1 to 4, 1 to 3, 1 or 2, bases have been deleted, substituted, or added, and proteins encoded by these DNAs inhibit the cell cycle.

A homologue of the BMI1 gene is a gene whose cDNA sequence is substantially the same as, for example, the nucleic acid sequence shown in SEQ ID NO: 2. A cDNA consisting of a sequence substantially the same as the nucleic acid sequence shown in SEQ ID NO: 2 refers to a DNA consisting of a sequence having not less than about 60%, preferably not less than about 70%, more preferably not less than about 80%, for example, 81%, 82%, 83%, 84%, 85%, 86%, 87%, 88%, 89%, further preferably about not less than 90%, for example, 91%, 92%, 93%, 94%, 95%, 96%, 97%, 98%, most preferably not less than about 99%, identity with the DNA consisting of the sequence represented by SEQ ID NO: 2, or a DNA that can hybridize under stringent conditions with DNA or RNA consisting of a sequence complementary to the nucleic acid sequence shown in SEQ ID NO: 2, wherein proteins encoded by these DNAs inhibit the cell cycle. Alternatively, the cDNA consisting of a sequence substantially the same as the nucleic acid sequence shown in SEQ ID NO: 2 refers to a DNA consisting of a sequence shown in SEQ ID NO: 2 in which one or more, for example, 1 to 10, preferably several, for example, 1 to 5, 1 to 4, 1 to 3, 1 or 2, bases have been deleted, substituted, or added, and proteins encoded by these DNAs inhibit the cell cycle.

A homologue of the BCL-XL gene is a gene whose cDNA sequence is substantially the same as, for example, the nucleic acid sequence shown in SEQ ID NO: 3. A cDNA consisting of a sequence substantially the same as the nucleic acid sequence shown in SEQ ID NO: 3 refers to a DNA consisting of a sequence having not less than about 60%, preferably not less than about 70%, more preferably not less than about 80%, for example, 81%, 82%, 83%, 84%, 85%, 86%, 87%, 88%, 89%, further preferably about not less than 90%, for example, 91%, 92%, 93%, 94%, 95%, 96%, 97%, 98%, most preferably not less than about 99%, identity with the DNA consisting of the sequence represented by SEQ ID NO: 3, or a DNA that can hybridize under stringent conditions with DNA or RNA consisting of a sequence complementary to the nucleic acid sequence shown in SEQ ID NO: 3, wherein proteins encoded by these DNAs inhibit the cell cycle. Alternatively, the cDNA consisting of a sequence substantially the same as the nucleic acid sequence shown in SEQ ID NO: 3 refers to a DNA consisting of a sequence shown in SEQ ID NO: 3 in which one or more, for example, 1 to 10, preferably several, for example, 1 to 5, 1 to 4, 1 to 3, 1 or 2, bases have been deleted, substituted, or added, and proteins encoded by these DNAs inhibit the cell cycle.

A homologue of the CDKN1A gene is a gene whose cDNA sequence is substantially the same as, for example, the nucleic acid sequence shown in SEQ ID NO: 4. A cDNA consisting of a sequence substantially the same as the nucleic acid sequence shown in SEQ ID NO: 4 refers to a DNA consisting of a sequence having not less than about 60%, preferably not less than about 70%, more preferably not less than about 80%, for example, 81%, 82%, 83%, 84%, 85%, 86%, 87%, 88%, 89%, further preferably about not less than 90%, for example, 91%, 92%, 93%, 94%, 95%, 96%, 97%, 98%, most preferably not less than about 99%, identity with the DNA consisting of the sequence represented by SEQ ID NO: 4, or a DNA that can hybridize under stringent conditions with DNA or RNA consisting of a sequence complementary to the nucleic acid sequence shown in SEQ ID NO: 4, wherein proteins encoded by these DNAs inhibit the cell cycle. Alternatively, the cDNA consisting of a sequence substantially the same as the nucleic acid sequence shown in SEQ ID NO: 4 refers to a DNA consisting of a sequence shown in SEQ ID NO: 4 in which one or more, for example, 1 to 10, preferably several, for example, 1 to 5, 1 to 4, 1 to 3, 1 or 2, bases have been deleted, substituted, or added, and proteins encoded by these DNAs inhibit the cell cycle.

The p53 gene is a gene whose cDNA sequence is substantially the same as, for example, the nucleic acid sequence shown in SEQ ID NO: 5. A cDNA consisting of a sequence substantially the same as the nucleic acid sequence shown in SEQ ID NO: 5 refers to a DNA consisting of a sequence having not less than about 60%, preferably not less than about 70%, more preferably not less than about 80%, for example, 81%, 82%, 83%, 84%, 85%, 86%, 87%, 88%, 89%, further preferably about not less than 90%, for example, 91%, 92%, 93%, 94%, 95%, 96%, 97%, 98%, most preferably not less than about 99%, identity with the DNA consisting of the sequence represented by SEQ ID NO: 5, or a DNA that can hybridize under stringent conditions with DNA consisting of a sequence complementary to the nucleic acid sequence shown in SEQ ID NO: 5, wherein proteins encoded by the DNA suppress cancer. Alternatively, the cDNA consisting of a sequence substantially the same as the nucleic acid sequence shown in SEQ ID NO: 5 refers to a DNA consisting of a sequence shown in SEQ ID NO: 5 in which one or more, for example, 1 to 10, preferably several, for example, 1 to 5, 1 to 4, 1 to 3, 1 or 2, bases have been deleted, substituted, or added, and proteins encoded by these DNAs suppress cancer.

Here, stringent conditions refer to hybridization conditions that can be easily determined by a person skilled in the art, and are empirical experimental conditions generally depending on the base length of the nucleic acid, washing temperature, and salt concentration. Generally, longer bases require higher temperatures for proper annealing and shorter bases require lower temperatures. Hybrid formation generally depends on the ability of complementary strand to reanneal in an environment slightly below the melting point thereof.

Specifically, examples of low stringency conditions include, for example, washing at a temperature of 37°C to 42°C in a 0.1×SSC, 0.1% SDS solution in the filter washing step after hybridization. In addition, examples of high stringency conditions include, for example, washing at 65°C in 5×SSC and 0.1% SDS in the washing step. Polynucleotides with high homology can be obtained by increasing the stringency conditions.

A method for producing CMPs or myeloid progenitor cells of the present embodiment includes a step of culturing (culture step) CMPs or myeloid progenitor cells obtained by the method for improving proliferative property of CMPs or myeloid progenitor cells according to the present embodiment described above.

The culture conditions for CMPs or myeloid progenitor cells can be appropriately determined by those skilled in the art depending on the type of cells and the state thereof. For example, the culture temperature can be set to about 35°C to about 42°C, about 36°C to about 40°C, or about 37°C to about 39°C, and the carbon dioxide concentration can be set to, for example, 5% CO₂, the oxygen concentration can be set to, for example, 20% 0₂. It may be static culture or shaking culture. The shaking speed in the case of shaking culture is also not particularly limited, and can be, for example, 10 rpm to 200 rpm, 30 rpm to 150 rpm, or the like.

The medium may be Iscove's Modified Dulbecco's Medium (IMDM) medium containing serum, insulin, transferrin, serine, thiolglycerol, ascorbic acid, TPO. In this case, the IMDM medium may further contain SCF and may further contain heparin. Furthermore, phorbol esters (e.g., phorbol-12-myristate-13-acetate; PMA) may also be added.

The cell culturing step can be performed in the presence or absence of feeder cells. In the present specification, the "feeder cell" refers to a cell that is co-cultured with a target cell in order to prepare an environment necessary for culturing the target cell to be proliferated or differentiated. The feeder cells may be allogenic cells or xenogeneic cells, as long as they can be distinguished from the target cells. The feeder cells may be cells treated with antibiotics or gamma rays to prevent proliferation, or may be untreated cells.

The medium may contain serum or plasma, or may be serum-free. When serum is used, human serum is preferred. As necessary, the medium may contain, for example, one or more substances such as albumin, insulin, transferrin, selenium, fatty acid, trace element, 2-mercaptoethanol, thiolglycerol, mono thioglycerol (MTG), lipid, amino acid (e.g., L-glutamine), ascorbic acid, heparin, non-essential amino acid, vitamin, growth factor, low-molecular-weight compound, antibiotic, antioxidant, pyruvic acid, buffering agent, inorganic salts, cytokine, and the like. Examples of the cytokine include vascular endothelial cell growth factor (VEGF), thrombopoietin (TPO), various TPO-like action substances, stem cell factor (SCF), erythropoietin (EPO), granulocyte colony stimulating factor (G-CSF), interleukin 3 (IL3), ITS (insulin-transferrin-selenite) supplement, ADAM (A Disintegrin And Metalloprotease) inhibitor, and the like.

Depending on the type of CMPs or myeloid progenitor cells to be cultured, it is preferable to add a combination of cytokines suitable for cell proliferation to the medium. For example, when culturing CMP, at least one, preferably all, cytokines selected from GM-CSF, G-CSF, IL-3, SCF, and TPO can be added to the medium in an amount sufficient to promote the proliferation of CMP. When culturing MEP, at least one, preferably all, cytokines selected from IL-3, SCF, and TPO can be added to the medium. When culturing GMP, at least one, preferably all, cytokines selected from SCF and GM-CSF can be added to the medium. When culturing macrophage progenitor cell, at least one, preferably all, cytokines selected from IL-1b, SCF and M-CSF can be added to the medium. When culturing dendritic cell progenitor cell, at least one, preferably all, cytokines selected from SCF, M-CSF and GM-CSF can be added to the medium. When culturing neutrophil progenitor cell, at least one, preferably all, cytokines selected from SCF and GM-CSF can be added to the medium. When culturing erythrocyte progenitor cell, at least one, preferably all, cytokines selected from SCF and EPO can be added to the medium.

The present invention provides CMPs or myeloid progenitor cells comprising a MYC family gene operably linked to the first exogenous promoter and BMI1 gene operably linked to the second exogenous promoter (hereinafter the cell of the present invention). The MYC family gene is preferably c-MYC. Examples of the myeloid progenitor cell include MEP, GMP, macrophage progenitor cell, dendritic cell progenitor cell, erythrocyte progenitor cell, neutrophil progenitor cell, and the like. The first exogenous promoter and the second exogenous promoter may be independently a constitutive promoter or a regulatable promoter, preferably a regulatable promoter. The regulatable promoter is preferably a drug-responsive promoter, more preferably a tetracycline-responsive promoter. The type of the first exogenous promoter and the second exogenous promoter may be the same or different, and they are preferably the same type of promoters. By using the same type of promoters, the MYC family gene and the BMI1 gene can be expressed synchronously, and the expression thereof can also be suppressed synchronously. The first exogenous promoter and the second exogenous promoter are preferably the same regulatable promoters (e.g., drug-responsive promoter), more preferably both tetracycline-responsive promoters. The first exogenous promoter and the second exogenous promoter may be each independently operably linked to the MYC family gene and the BMI1 gene, or the MYC family gene and the MYC family gene may be operably linked to one exogenous promoter. In this case, the MYC family gene and the BMI1 gene are linked via an intervening sequence such as IRES, thereby enabling bicistronic expression under the control of one exogenous promoter. The MYC family gene operably linked to the first exogenous promoter and the BMI1 gene operably linked to the second exogenous promoter may be integrated into the genome of the CMP or myeloid progenitor cell, or, it may be present in an expression vector introduced into the CMPs or myeloid progenitor cells. Preferably, the MYC family gene operably linked to the first exogenous promoter and the BMI1 gene operably linked to the second exogenous promoter are integrated into the genome of the CMP or myeloid progenitor cell.

When using a tetracycline-responsive promoter as the first exogenous promoter and/or the second exogenous promoter, in order to enable tetracycline-dependent expression control, the cell of the present invention preferably further has an rtTA gene or tTA gene operably linked to a third exogenous promoter. The third exogenous promoter may be a constitutive promoter or regulatable promoter, preferably a constitutive promoter. The rtTA gene or tTA gene operably linked to the third exogenous promoter may be integrated into the genome of the CMP or myeloid progenitor cell, or may be present in an expression vector introduced into the genome of CMP or myeloid progenitor cell. Preferably, the rtTA gene or tTA gene operably linked to the third exogenous promoter is integrated into the genome of the CMP or myeloid progenitor cell.

The cell of the present invention expresses the MYC family gene (e.g., c-Myc gene) and the BMI1 gene in amounts that can promote the proliferation of a CMP or a myeloid progenitor cell in vitro, when the cell is cultured under conditions where CMP or myeloid progenitor cells can be proliferated. The amounts of the MYC family gene (e.g., c-Myc) and BMI1 gene that can promote the proliferation of CMP or myeloid progenitor cells in vitro mean amounts of MYC family gene and BMI1 gene that significantly increase the rate of proliferation of CMPs or myeloid progenitor cells expressing such amounts of the MYC family gene and BMI1 gene, as compared with CMP or myeloid progenitor cells prepared in the same manner as the above-mentioned cells except that they do not express the MYC family gene and BMI1 gene.

From the viewpoint of enhancing proliferation ability, the cell of the present invention may further have BCL-XL gene operably linked to a fourth exogenous promoter. When the cell is cultured under conditions where the fourth exogenous promoter operates, it is expected that the BCL-XL gene is expressed and the proliferation of the cell of the present invention is further promoted. The fourth exogenous promoter may be independently a constitutive promoter or a regulatable promoter, preferably a regulatable promoter. The regulatable promoter is preferably a drug-responsive promoter, more preferably a tetracycline-responsive promoter. The type of the fourth exogenous promoter may be the same as or different from type of the first exogenous promoter and/or the second exogenous promoter, and they are preferably the same type of promoters. By using the same type of promoters, the MYC family gene, BMI1 gene, and BCL-XL gene can be expressed synchronously, and the expression thereof can also be suppressed synchronously. The first exogenous promoter, the second exogenous promoter, and the fourth exogenous promoter are preferably the same regulatable promoters (e.g., drug-responsive promoters), more preferably all tetracycline-responsive promoters. The fourth exogenous promoter may be operably linked to the BCL-XL gene independently from the first and the second exogenous promoters, the MYC family gene and BCL-XL gene may be operably linked to the first exogenous promoter, the BMI1 gene and ·BR>ACL-XL gene may be operably linked to the second exogenous promoter, or the MYC family gene, BMI1 gene and BCL-XL gene may be operably linked to one exogenous promoter. By linking plural genes via an intervening sequence such as RES or the like, a bicistronic expression can be performed under the control of one exogenous promoter. The BCL-XL gene operably linked to the fourth exogenous promoter may be integrated into the genome of the CMP or myeloid progenitor cell, or may also be present in an expression vector introduced into CMPs or myeloid progenitor cells. Preferably, the BCL-XL gene operably linked to the fourth exogenous promoter is integrated into the genome of the CMP or myeloid progenitor cell.

From the viewpoint of enhancing proliferation ability, the cell of the present invention may further have a nucleic acid encoding an expression suppressor nucleic acid (e.g., siRNA, shRNA, antisense nucleic acid) for the CDKN1A gene operably linked to a fifth exogenous promoter and/or a nucleic acid encoding an expression suppressor nucleic acid for the p53 gene operably linked to a sixth exogenous promoter. When the cell is cultured under conditions where the fifth exogenous promoter and/or the sixth exogenous promoter operate, it is expected that an expression suppressor nucleic acid for the CDKN1A gene and/or an expression suppressor nucleic acid for the p53 gene are expressed, and the proliferation of the cell of the present invention is further promoted. The fifth exogenous promoter and the sixth exogenous promoter may be independently constitutive promoters or regulatable promoters, preferably constitutive promoters. The constitutive promoter is preferably a pol III promoter such as H1 promoter. The type of the fifth exogenous promoter may be the same as or different from the type of the sixth exogenous promoter. The nucleic acid encoding an expression suppressor nucleic acid for the CDKN1A gene operably linked to the fifth exogenous promoter and the nucleic acid encoding an expression suppressor nucleic acid for the p53 gene operably linked to the sixth exogenous promoter may be integrated into the genome of CMP or myeloid progenitor cell, or may be present in an expression vector introduced into CMPs or myeloid progenitor cells. Preferably, they are integrated into the genome of the CMP or myeloid progenitor cell.

In one embodiment, the cell of the present invention is a CMP or a myeloid progenitor cell (e.g., MEP, GMP, macrophage progenitor cell, dendritic cell progenitor cell, erythrocyte progenitor cell, neutrophil progenitor cell) having a MYC family gene operably linked to the first exogenous promoter, BMI1 gene operably linked to the second exogenous promoter, and BCL-XL gene operably linked to the fourth exogenous promoter. When a tetracycline-responsive promoter is used as the first exogenous promoter and/or the second exogenous promoter, the cell of the present invention further optionally has rtTA gene or tTA gene operably linked to the third exogenous promoter.

In one embodiment, the cell of the present invention is a CMP or a myeloid progenitor cell (e.g., MEP, GMP, macrophage progenitor cell, dendritic cell progenitor cell, erythrocyte progenitor cell, neutrophil progenitor cell) having a MYC family gene operably linked to the first exogenous promoter,
BMI1 gene operably linked to the second exogenous promoter, a nucleic acid encoding expression suppressor nucleic acid for CDKN1A gene operably linked to the fifth exogenous promoter, and
a nucleic acid encoding expression suppressor nucleic acid for p53 gene operably linked to the sixth exogenous promoter. When a tetracycline-responsive promoter is used as the first exogenous promoter and/or the second exogenous promoter, the cell of the present invention further optionally has rtTA gene or tTA gene operably linked to the third exogenous promoter.

In one embodiment, the cell of the present invention is a CMP or a myeloid progenitor cell (e.g., MEP, GMP, macrophage progenitor cell, dendritic cell progenitor cell, erythrocyte progenitor cell, neutrophil progenitor cell) having a MYC family gene operably linked to the first exogenous promoter,
BMI1 gene operably linked to the second exogenous promoter, BCL-XL gene operably linked to the fourth exogenous promoter, a nucleic acid encoding expression suppressor nucleic acid for CDKN1A gene operably linked to the fifth exogenous promoter, and
a nucleic acid encoding expression suppressor nucleic acid for p53 gene operably linked to the sixth exogenous promoter. When a tetracycline-responsive promoter is used as the first exogenous promoter and/or the second exogenous promoter, the cell of the present invention further optionally has rtTA gene or tTA gene operably linked to the third exogenous promoter.

The cell of the present invention can be obtained by the aforementioned method for improving proliferative property of a CMP or a myeloid progenitor cell, or method for producing CMPs or myeloid progenitor cells of the present invention.

In addition, the present invention provides a cell population containing the above-mentioned cell of the present invention (to be referred to as the cell population of the present invention). The cell population abundantly contains the above-mentioned cell of the present invention, and the ratio of the cell of the present invention contained in the whole cell population is, for example, not less than 10%, not less than 20%, not less than 30%, not less than 40%, not less than 50%, not less than 60%, not less than 70%, not less than 80%, not less than 90%, not less than 95% (e.g., 100%). Such cell population abundantly containing the cell of the present invention can be obtained by extracting the cells of interest in a specific stage of differentiation (CMPs or myeloid progenitor cells (e.g., MEP, GMP, macrophage progenitor cell, dendritic cell progenitor cell, erythrocyte progenitor cell, neutrophil progenitor cell)) from a cell population subjected to the above-mentioned method of the present invention. In a preferred embodiment, the cell population of the present invention abundantly contains the cell of the present invention in a specific stage of differentiation. In one embodiment, the ratio of the cell of the present invention contained in the whole cell population is (the cell is CMP) not less than 10%, not less than 20%, not less than 30%, not less than 40%, not less than 50%, not less than 60%, not less than 70%, not less than 80%, not less than 90%, not less than 95% (e.g., 100%). In one embodiment, the ratio of the cell of the present invention contained in the whole cell population is (the cell is MEP) not less than 10%, not less than 20%, not less than 30%, not less than 40%, not less than 50%, not less than 60%, not less than 70%, not less than 80%, not less than 90%, not less than 95% (e.g., 100%). In one embodiment, the ratio of the cell of the present invention contained in the whole cell population is (the cell is GMP) not less than 10%, not less than 20%, not less than 30%, not less than 40%, not less than 50%, not less than 60%, not less than 70%, not less than 80%, not less than 90%, not less than 95% (e.g., 100%). In one embodiment, the ratio of the cell of the present invention contained in the whole cell population is (the cell is macrophage progenitor cell) not less than 10%, not less than 20%, not less than 30%, not less than 40%, not less than 50%, not less than 60%, not less than 70%, not less than 80%, not less than 90%, not less than 95% (e.g., 100%). In one embodiment, the ratio of the cell of the present invention contained in the whole cell population is (the cell is dendritic cell progenitor cell) not less than 10%, not less than 20%, not less than 30%, not less than 40%, not less than 50%, not less than 60%, not less than 70%, not less than 80%, not less than 90%, not less than 95% (e.g., 100%). In one embodiment, the ratio of the cell of the present invention contained in the whole cell population is (the cell is erythrocyte progenitor cell) not less than 10%, not less than 20%, not less than 30%, not less than 40%, not less than 50%, not less than 60%, not less than 70%, not less than 80%, not less than 90%, not less than 95% (e.g., 100%). In one embodiment, the ratio of the cell of the present invention contained in the whole cell population is (the cell is neutrophil progenitor cell) not less than 10%, not less than 20%, not less than 30%, not less than 40%, not less than 50%, not less than 60%, not less than 70%, not less than 80%, not less than 90%, not less than 95% (e.g., 100%). Such cell population abundantly containing the cell of the present invention in a specific stage of differentiation can be obtained by isolating and extracting the cells of interest in a specific stage of differentiation from a cell population subjected to the above-mentioned method of the present invention by using a cell sorter or the like and an antibody against a cell surface marker specifically expressed in the cell in the differentiation stage.

The cell of the present invention and the cell population of the present invention can be obtained by the aforementioned method for improving proliferative property of a CMP or a myeloid progenitor cell or method for producing CMPs or myeloid progenitor cells of the present invention.

Furthermore, the present invention provides a cell preparation (to be referred to as the cell preparation of the present invention) containing the above-mentioned cell population of the present invention. The cell preparation of the present invention can be adjusted by suspending the above-mentioned cell population of the present invention in an appropriate physiological aqueous solution (e.g., physiological saline, isotonic solution containing glucose and other auxiliary agents, liquid medium). The physiological aqueous solution may contain buffering agent (e.g., phosphate buffer, sodium acetate buffer), soothing agent (e.g., hydrochloric acid lidocain, procaine hydrochloride, etc.), stabilizer (e.g., human serum albumin, polyethylene glycol, etc.), preservative (e.g., sodium benzoate, benzalkonium chloride, etc.), antioxidant (e.g., ascorbic acid, sodium edentate, etc.), and the like. In the cell preparation, the above-mentioned cell population of the present invention is suspended at a cell concentration of, for example, 1.0×10¹ to 1.0×10¹² cells/mL.

Depending on the type of CMPs or myeloid progenitor cells to be contained in the cell preparation, a combination of cytokines suitable for proliferation of the cell may be added to the cell preparation. For example, in the case of a cell preparation containing CMP, at least one, preferably all, cytokines selected from GM-CSF, G-CSF, IL-3, SCF, and TPO can be added to the cell preparation. In the case of a cell preparation containing MEP, at least one, preferably all, cytokines selected from IL-3, SCF, and TPO can be added to the cell preparation. In the case of a cell preparation containing GMP, at least one, preferably all, cytokines selected from SCF and GM-CSF can be added to the cell preparation. In the case of a cell preparation containing macrophage progenitor cell, at least one, preferably all, cytokines selected from IL-1b, SCF, and M-CSF can be added to the cell preparation. In the case of a cell preparation containing dendritic cell progenitor cell, at least one, preferably all, cytokines selected from SCF, M-CSF, and GM-CSF can be added to the cell preparation. In the case of a cell preparation containing neutrophil progenitor cell, at least one, preferably all, cytokines selected from SCF and GM-CSF can be added to the cell preparation. In the case of a cell preparation containing erythrocyte progenitor cell, at least one, preferably all, cytokines selected from SCF and EPO can be added to the cell preparation.

CMPs or myeloid progenitor cells may have freeze-thaw resistance that maintains cell proliferation ability and differentiation ability even after thawing after cryopreservation. Therefore, CMP-lineage differentiated cells can be produced by cryopreserving CMPs or myeloid progenitor cells, dissolving them if necessary, and subjecting them to differentiation-inducing culture. Therefore, by using the above-mentioned cell of the present invention, there is no need to start from the first step of a series of operations for producing CMP-lineage differentiated cells (e.g., macrophage, dendritic cell, erythrocyte, neutrophil, and the like) from pluripotent stem cells such as ES cells and iPS cells. In other words, by preparing a large amount of the cell of the present invention as a raw material and cryopreserving them as necessary, the production process can be streamlined and made more efficient, and a system for rapidly supplying various CMP-lineage differentiated cells such as macrophage, dendritic cell, erythrocyte, neutrophil, and the like can be constructed. Therefore, in one embodiment, the cell preparation of the present invention is a frozen cell preparation containing the above-mentioned frozen cell population of the present invention. When producing a frozen cell preparation using the cells of the present invention, it can be composed of the above-mentioned cell population of the present invention and a cryopreservation solution, and other additives may also be added to the composition as necessary. As the cryopreservation solution, a freezing solution containing DMSO or the like can be used. Specifically, Cell Banker (Nippon Zenyaku Kogyo Co., Ltd.), Van Bunker (Nippon Genetics Co., Ltd.), TC Protector (DS Pharma Biomedical Co., Ltd.), Albumin-added CP-1 (Kyokuto Pharmaceutical Kogyo Co., Ltd.), and the like can be mentioned.

The method for producing CMP-lineage differentiated cells according to this embodiment includes a step of differentiating a CMP or a myeloid progenitor cell (that is, the cell of the present invention) obtained by the aforementioned method for improving proliferative property of a CMP or a myeloid progenitor cell or method for producing CMPs or myeloid progenitor cells. The CMP-lineage differentiated cells are monocytes such as macrophage, dendritic cell, and the like, granulocytes such as neutrophil, basophil, and the like, erythroblasts, erythrocytes, and the like, which are differentiated from CMPs or myeloid progenitor cells, and are distinguished from myeloid progenitor cell.

As a method for differentiating CMPs or myeloid progenitor cells, known methods for inducing differentiation into monocytes such as macrophage and dendritic cell, granulocytes such as neutrophil and basophil, erythroblasts, or erythrocytes can be appropriately selected, including the above-mentioned culture methods. For example, the drug used in the step of forced expression of each gene, such as tetracycline or doxycycline, is added to the medium, the forced expression is suppressed by removing same from the medium, and the cells may be subsequently cultured.

The CMP-lineage differentiated cell may contain monocyte, granulocyte, or erythroblast, and may contain monocyte or granulocyte. In a method for controlling CMPs to differentiate into specific cells, suitable known media or media analogous thereto can be used as appropriate. For example, in the production method of the CMP-lineage differentiated cells according to this embodiment, by controlling cytokine conditions included in the medium, the type of cells to be differentiated can be controlled. Specifically, CMP-lineage differentiated cells containing monocyte, granulocyte, or erythroblast can be obtained by culturing using cytokines, thrombopoietin (TPO), stem cell factor (SCF), erythropoietin (EPO), granulocyte colony stimulating factor (G-CSF), and interleukin 3 (IL3). In addition, CMP-lineage differentiated cells containing monocyte or granulocyte can be obtained using cytokines, thrombopoietin (TPO), stem cell factor (SCF), granulocyte macrophage colony stimulating factor (GM-CSF), granulocyte colony stimulating factor (G-CSF), and interleukin 3 (IL3). Specifically, cytokine conditions for differentiation into various cell types include those shown in the Examples.

For example, cultivation of macrophage progenitor cells in which a MYC family gene (e.g., c-Myc gene) and BMI1 gene (optionally further BCL-XL gene) are forcibly expressed under the control of drug (e.g., tetracycline, doxycycline)-responsive promoter by the addition of said drug and the expression of the CDKN1A gene and/or p53 gene, or the function of expression products thereof, is optionally suppressed, under macrophage differentiation conditions (e.g., in the presence of SCF, M-CSF, IL-1b) results in good proliferation of macrophage progenitor cells while maintaining their differentiation stage. Cultivation of the cells after suppressing the forced expression of the MYC family gene (e.g., c-Myc gene) and the BMI1 gene (optionally further the BCL-XL gene) in the cells by removal of the drug under macrophage differentiation conditions (e.g., in the presence of SCF, M-CSF, IL-1b) results in suppression of cell proliferation and promoted differentiation into macrophages and maturation thereof. Cultivation of dendritic cell progenitor cells in which the MYC family gene (e.g., c-Myc gene) and the BMI1 gene (optionally further BCL-XL gene) are forcibly expressed under the control of drug (tetracycline, doxycycline)-responsive promoter by the addition of said drug and the expression of the CDKN1A gene and/or p53 gene, or the function of expression products thereof, is optionally suppressed, under dendritic cell differentiation conditions (e.g., in the presence of SCF, M-CSF, GM-CSF) results in good proliferation of dendritic cell progenitor cells while maintaining their differentiation stage. Cultivation of the cells after suppressing the forced expression of the MYC family gene (e.g., c-Myc gene) and the BMI1 gene (optionally further BCL-XL gene) in the cells by removal of the drug under dendritic cell differentiation conditions (e.g., in the presence of SCF, M-CSF, GM-CSF) results in suppression of cell proliferation and promoted differentiation into dendritic cells and maturation thereof. Cultivation of erythrocyte progenitor cells in which the MYC family gene (e.g., c-Myc gene) and the BMI1 gene (optionally further the BCL-XL gene) under the control of drug (tetracycline, doxycycline)-responsive promoter by the addition of said drug and the expression of the CDKN1A gene and/or p53 gene, or the function of expression products thereof, is optionally suppressed, under erythrocyte differentiation conditions (e.g., in the presence of SCF, EPO) results in good proliferation of erythrocyte progenitor cells while maintaining their differentiation stage. Cultivation of the cells after suppressing the forced expression of the MYC family gene (e.g., c-Myc gene) and the BMI1 gene (optionally further the BCL-XL gene) in the cells by removal of the drug under erythrocyte differentiation conditions (e.g., in the presence of SCF, EPO) results in suppression of cell proliferation and promoted differentiation into erythrocytes and maturation thereof.

The proliferation promoting agent for CMPs or myeloid progenitor cells according to the present embodiment contains, as an active ingredient, a molecule that forcibly expresses the MYC family gene and the BMI1 gene, and optionally contains, as an active ingredient, a molecule that forcibly expresses the BCL-XL gene, or contains, as an active ingredient, a molecule that suppresses the expression of the CDKN1A gene or p53 gene or the function of expression products thereof. Moreover, myelocytic precursor cell is a precursor cell of macrophage, dendritic cell, granulocyte, erythroblast, or erythrocyte.

The pharmaceutical composition according to the present embodiment includes CMPs or myeloid progenitor cells obtained by the aforementioned method for improving proliferative property of CMP or myeloid progenitor cell or method for producing CMP or myeloid progenitor cells, or CMP-lineage differentiated cell obtained by the aforementioned method for producing CMP-lineage differentiated cell.

The CMP or myeloid progenitor cell obtained by the aforementioned method for improving proliferative property of CMP or myeloid progenitor cell or method for producing CMP or myeloid progenitor cells, and CMP-lineage differentiated cell obtained by the aforementioned method for producing CMP-lineage differentiated cell are cells that control the innate immune system and can be used to eliminate tumors, degenerated cells, and pathogenic organisms. Therefore, the pharmaceutical composition according to the present embodiment can be used for removing tumors, degenerated cells, infectious pathogenic organisms, and the like. Specifically, by introducing receptors that target foreign body-specific antigens in the normal body and genetically modifying them to increase cytotoxicity to target cells, the composition can be used as an immune cell preparation for blood transfusion specific to each disease. In addition, it is also used as an immunological preparation, an anticancer agent, an antiallergic preparation, or a pharmaceutical composition for antiarteriosclerosis. The aforementioned CMPs, myeloid progenitor cells, or CMP-lineage differentiated cells are mixed with a pharmaceutically acceptable carrier according to conventional means and are manufactured as a parenteral preparation such as injection, suspension, drip transfusion, or the like. Examples of the pharmaceutically acceptable carrier which may be contained in the parenteral preparation include, but are not limited to, aqueous solutions for injection such as isotonic solutions (e.g., D-sorbitol, D-mannitol, sodium chloride, etc.) containing saline, glucose, or other auxiliary agents, and the like. The pharmaceutical composition of the present invention may be blended with, for example, buffering agent (e.g., phosphate buffer, sodium acetate buffer), soothing agent (e.g., hydrochloric acid lidocain, procaine hydrochloride, etc.), stabilizer (e.g., human serum albumin, polyethylene glycol, etc.), preservative (e.g., sodium benzoate, benzalkonium chloride, etc.), antioxidant (e.g., ascorbic acid, sodium edentate, etc.), and the like. When the pharmaceutical composition of the present invention is formulated as an aqueous suspension, the aforementioned CMP, myeloid progenitor cells, or CMP-lineage differentiated cells may be suspended in the above-mentioned aqueous solution at a cell concentration of, for example, about 1.0×10² to about 1.0×10¹² cells/mL.

The method for treating or preventing a disease according to this embodiment includes administering CMPs or myeloid progenitor cells obtained by the aforementioned method for improving proliferative property of a CMP or a myeloid progenitor cell or method for producing CMPs or myeloid progenitor cells, or CMP-lineage differentiated cells obtained by the aforementioned method for producing CMP-lineage differentiated cells, or the pharmaceutical composition according to the present embodiment, to a patient in need thereof. Examples of the disease include, but are not particularly limited to, diseases related to tumors/degenerated cells or infectious pathogenic organisms, inflammatory/allergic diseases (autoimmune diseases, chronic inflammatory diseases such as arteriosclerosis), and the like. The administration method is not particularly limited, but injection is preferred, and examples include intravenous administration, intraperitoneal administration, and the like. The dosage of the agent of the present invention varies depending on the subject of administration, treatment target site, symptoms, administration method, and the like. For example, in the case of intravenous administration, the dosage for a patient (body weight of 60 kg) is generally about 1.0×10⁶ to about 1.0×10¹¹ cells in an amount of human myeloid blood cells and it can be administered about 2 to 3 times a week for about 2 to 3 weeks or more.

The kit according to the present embodiment contains the above-mentioned proliferation promoting agent for a CMP or a myeloid progenitor cell, and is used in the diagnosis of diseases related to tumors, degenerated cells, infectious pathogenic organisms, and inflammatory/allergic diseases (autoimmune diseases, chronic inflammatory diseases such as arteriosclerosis, etc.).

The kit may contain reagents, carriers, and additives according to the use thereof, and may further contain buffers, containers, instruction manual, and the like. The specific form of the kit is, for example, it contains a proliferation promoting agent for a CMP or a myeloid progenitor cell, and a diagnostic chip used for specific cell types such as neutrophil, macrophage, and dendritic cell, which are the main causal cells of inflammatory/allergic diseases (autoimmune diseases, chronic inflammatory diseases such as arteriosclerosis, etc.).

### (2) Methodology 2

In a further aspect, the present invention provides a method for improving proliferative property of CMPs or myeloid progenitor cells, including a step of forcibly expressing BCL-XL gene in the CMPs or myeloid progenitor cells (hereinafter to be referred to as "the method 2 of the present invention"). The myeloid progenitor cell is preferably a GMP, a macrophage progenitor cell, or a dendritic cell progenitor cell. By forcibly expressing the BCL-XL gene, it is expected that the proliferative property of CMPs or myeloid progenitor cells is improved, and an immortalized cell line that proliferates infinitely can be obtained.

The method 2 of the present invention may further include a step of extracting (isolating or purifying) cells (CMPs or myeloid progenitor cells) at a specific desired stage of differentiation. The cells (CMPs or myeloid progenitor cells) at a specific stage of differentiation may be extracted before or after forcibly expressing the BCL-XL gene. In one embodiment, this extraction step is performed before forcibly expressing the BCL-XL gene, and the BCL-XL gene are forcibly expressed in the extracted cells (CMPs or myeloid progenitor cells) at a specific stage of differentiation. In another embodiment, a cell population containing cells (CMPs or myeloid progenitor cells) at a specific desired stage of differentiation in which the BCL-XL gene has been forcibly expressed is prepared, and then the cells (CMPs or myeloid progenitor cells) at a specific desired stage of differentiation are extracted from the cell population. The BCL-XL gene may continuously be expressed forcibly in these extracted cells at a specific stage of differentiation (CMPs or myeloid progenitor cells). By extracting the cells of interest and applying the method of the present invention to the extracted cells, or by extracting the cells of interest from a cell population to which the method of the present invention has been applied and continuously culturing the cells, the cell type of interest can be efficiently proliferated. From the aspect of efficiently improving the proliferative property of the extracted cell type, the cell to be extracted is preferably only the cell line of CMP or only a single type of cell line of myeloid progenitor cell, or may be a cell population of a mixture of two or more types of these cells. The cell to be extracted is preferably a CMP, a GMP, a macrophage progenitor cell, or a dendritic cell progenitor cell. Extraction of the cells of interest can be performed by the methods described in Methodology 1. The cells of interest may be isolated such that the percentage of the cells of interest in the cell population after the extraction operation is, for example, not less than 10%, not less than 20%, not less than 30%, not less than 40%, not less than 50%, not less than 60%, not less than 70%, not less than 80%, not less than 90%, not less than 95% (e.g., 100%). Single cells of the cells of interest may be isolated.

The method 2 of the present invention may further include a step of forcibly expressing a MYC family gene (preferably c-Myc) and BMI1 gene in CMPs or myeloid progenitor cells. By expressing the MYC family gene (preferably c-Myc) and the BMI1 gene in addition to the BCL-XL, further promotion of proliferation of CMPs or myeloid progenitor cells can be expected is expected. The period of the forced expression of the MYC family gene (preferably c-Myc) and the BMI1 gene can be appropriately determined by those of ordinary skill in the art.

The forced expression of the MYC family gene, BMI1 gene, and BCL-XL gene may be performed simultaneously or successively. For example, the MYC family gene and the BMI1 gene may be forcibly expressed and the BCL-XL gene may successively be expressed forcibly to obtain CMPs or myeloid progenitor cells with improved proliferation potency. In addition, CMP or myeloid progenitor cell with improved proliferation potency can also be obtained by forcibly expressing simultaneously the MYC family gene, the BMI1 gene, and the BCL-XL gene. When maintaining proliferation of CMPs or myeloid progenitor cells, it is preferable to maintain forced expression of the MYC family gene, BMI1 gene, and BCL-XL gene during the culture period.

The MYC family gene, BMI1 gene, and BCL-XL gene promote proliferation of CMPs or myeloid progenitor cells, but can inhibit terminal differentiation of CMP-lineage differentiated cells (e.g., macrophage, dendritic cell, neutrophil, erythrocyte). Thus, expression of these genes may be suppressed before entering the terminal differentiation step. Suppressing the expression of these genes in CMPs or myeloid progenitor cells facilitates the induction of functional, more mature CMP-lineage differentiated cells (e.g., macrophage, dendritic cell, neutrophil, erythrocyte).

Genes such as the MYC family gene, BMI1 gene, BCL-XL gene, and the like can be forcibly expressed in cells according to the methods described in Methodology 1. CMPs or myeloid progenitor cells may be transfected with an expression vector of the desired gene (e.g., BCL-XL gene, optionally further a MYC family gene and BMI1 gene), or CMPs or myeloid progenitor cells may be induced from pluripotent stem cells (e.g., ES cells, iPS cells), hematopoietic progenitor cells, or hematopoietic endothelial cells that have been incorporated in advance with expression cassettes for the desired gene (e.g., the BCL-XL gene, optionally further the MYC family gene and BMI1 gene), and the gene may be forcibly expressed at this stage. Alternatively, the CMPs or myeloid progenitor cells may be induced by differentiating pluripotent stem cells (e.g., ES cells, iPS cells), hematopoietic progenitor cells, or hematopoietic endothelial cells that have been incorporated in advance with expression cassettes for the desired gene (e.g., the BCL-XL gene, optionally further the MYC family gene and BMI1 gene), and in which the said gene is forcibly expressed. The MYC family gene, BMI1 gene, and BCL-XL gene, which are the target genes for controlling expression, may be inserted in separate vectors or in the same vector.

Suppression of the expression of the MYC family gene, BMI1 gene, BCL-XL gene, and the like in cells may be performed according to the methods described in Methodology 1.

The method 2 of the present invention may include a step of suppressing the expression of CDKN1A gene and/or p53 gene or the function of expression products thereof, in the CMPs or myeloid progenitor cells. In method 2 of the present invention, suppressing the expression of the CDKN1A gene and/or p53 gene, or the function of expression products thereof, can be expected to further improve the proliferation of CMPs or myeloid progenitor cells.

It is preferable to suppress the expression of not only the CDKN1A gene but the p53 gene, or the function of expression products thereof.

Suppression of the expression of the above-mentioned respective genes or the function of expression products thereof can be performed according to the methods described in Methodology 1.

Suppression of the expression of the CDKN1A gene and/or the p53 gene is preferably performed, similar to Methodology 1, by introducing, into the cells, expression vectors expressing the expression suppressor nucleic acids for the respective genes. CMPs or myeloid progenitor cells may be transfected with an expression vector (e.g., viral vector) of an expression suppressor nucleic acid for the desired gene (e.g., CDKN1A gene, p53 gene), or CMPs or myeloid progenitor cells may be induced from pluripotent stem cells (e.g., ES cells, iPS cells), hematopoietic progenitor cells, or hematopoietic endothelial cells that have been incorporated in advance with expression cassettes of an expression suppressor nucleic acid for the desired gene (e.g., CDKN1A gene, p53 gene), and the siRNA, shRNA, or antisense nucleic acid may be forcibly expressed at this stage. Alternatively, CMPs or myeloid progenitor cells may be induced by differentiating pluripotent stem cells (e.g., ES cells, iPS cells), hematopoietic progenitor cells, or hematopoietic endothelial cells that have been incorporated in advance with expression cassette for the expression suppressor nucleic acid for the desired gene (e.g., CDKN1A gene, p53 gene), and in which the said expression suppressor nucleic acid is forcibly expressed. When the expression suppressor nucleic acid is expressed using an expression vector, a nucleic acid (e.g., DNA) encoding the expression suppressor nucleic acid can be linked downstream of an appropriate promoter, inserted into the expression vector, and then introduced into the cell to express the expression suppressor nucleic acid of interest. The promoter can be an exogenous promoter. The exogenous promoter may be a constitutive promoter or a regulatable promoter, preferably a constitutive promoter. The nucleic acid encoding the expression suppressor nucleic acid for the CDKN1A gene and the nucleic acid encoding the expression suppressor nucleic acid for the p53 gene may be inserted into separate expression vectors, or may be inserted into the same expression vector.

In this aspect, suppression of the expression of the CDKN1A gene and/or p53 gene or the function of expression products thereof may be performed simultaneously with the forced expression of any of the MYC family gene, BMI1 gene, and BCL-XL gene. It is preferably performed simultaneously with the forced expression of the BCL-XL gene or thereafter. For example, it can be performed after confirmation of a decrease in the cell proliferation. In one embodiment, the cell proliferation rate at a certain time point is compared to the most recent cell proliferation rate (for example, when cell proliferation is confirmed every week, the cell proliferation rate of a certain week is compared with the proliferation rate of one week before), and it can be performed after confirmation of the state in which the proliferation rate has decreased to 1/2 or less. In one embodiment, in CMPs or myeloid progenitor cells, the MYC family gene (e.g., c-Myc gene) and the BMI1 gene are forcibly expressed and, in parallel, the expression of the CDKN1A gene and/or p53 gene or the function of expression products thereof is suppressed.

The present invention also provides a method for producing CMPs or myeloid progenitor cells, including a step of culturing CMPs or myeloid progenitor cells obtained in the above-mentioned method 2 of the present invention (culture step) (hereinafter to be referred to as the production method 2 of the present invention).

The culture conditions for CMPs or myeloid progenitor cells are as described in Methodology 1. As described in Methodology 1, a combination of cytokines suitable for cell proliferation can be added to the medium, depending on the type of CMPs or myeloid progenitor cells to be cultured.

The present invention provides a CMP or a myeloid progenitor cell having BCL-XL gene operably linked to a fourth exogenous promoter (hereinafter the cell 2 of the present invention). The myeloid progenitor cell is preferably a GMP, a macrophage progenitor cell, or a dendritic cell progenitor cell. The explanation on the terms for the fourth exogenous promoter follows the part described in Methodology 1. The BCL-XL gene operably linked to the fourth exogenous promoter may be integrated into the genome of the CMP or myeloid progenitor cell or it may be present in an expression vector introduced into the CMPs or myeloid progenitor cells. Preferably, the BCL-XL gene operably linked to the fourth exogenous promoter is integrated into the genome of the CMP or myeloid progenitor cell.

When using a tetracycline-responsive promoter as the fourth exogenous promoter, in order to enable tetracycline-dependent expression control, the cell of the present invention preferably further has an rtTA gene or tTA gene operably linked to a third exogenous promoter. The third exogenous promoter may be a constitutive promoter or regulatable promoter, preferably a constitutive promoter. The rtTA gene or tTA gene operably linked to the third exogenous promoter may be integrated into the genome of the CMP or myeloid progenitor cell, or may be present in an expression vector introduced into the CMPs or myeloid progenitor cells. Preferably, the rtTA gene or tTA gene operably linked to the third exogenous promoter is integrated into the genome of the CMP or myeloid progenitor cell.

From the viewpoint of enhancing proliferation ability, the cell 2 of the present invention may further have a MYC family gene operably linked to a first exogenous promoter and BMI1 gene operably linked to a second exogenous promoter. The MYC family gene is preferably c-MYC. The explanation on the terms for the first exogenous promoter and the second exogenous promoter follows the part described in Methodology 1. The MYC family gene operably linked to the first exogenous promoter and the BMI1 gene operably linked to the second exogenous promoter may be integrated into the CMP or myeloid progenitor cell, or they may also be present in an expression vector introduced into the CMPs or myeloid progenitor cells. Preferably, the MYC family gene operably linked to the first exogenous promoter and the BMI1 gene operably linked to the second exogenous promoter are integrated into the genome of the CMP or myeloid progenitor cell.

The type of the first exogenous promoter and/or the second exogenous promoter may be the same as or different from that of the fourth exogenous promoter, and they are preferably the same type of promoters. By using the same type of promoters, the MYC family gene, BMI1 gene, and BCL-XL gene can be expressed synchronously, and the expression thereof can also be suppressed synchronously. The first exogenous promoter, the second exogenous promoter, and the fourth exogenous promoter are preferably the same regulatable promoters (e.g., drug-responsive promoters), more preferably all tetracycline-responsive promoters. The fourth exogenous promoter may be operably linked to the BCL-XL gene independently from the first and the second exogenous promoters, the MYC family gene and the BCL-XL gene may be operably linked to the fourth exogenous promoter, the BMI1 gene and the BCL-XL gene may be operably linked to the fourth exogenous promoter, or the MYC family gene, BMI1 gene and BCL-XL gene may be operably linked to one exogenous promoter.

When using a tetracycline-responsive promoter as at least one of the first, the second, and the fourth exogenous promoters, in order to enable tetracycline-dependent expression control, the cell 2 of the present invention preferably further has an rtTA gene or tTA gene operably linked to a third exogenous promoter. The third exogenous promoter may be a constitutive promoter or regulatable promoter, preferably a constitutive promoter. The rtTA gene or tTA gene operably linked to the third exogenous promoter may be integrated into the genome of the CMP or myeloid progenitor cell, or may be present in an expression vector introduced into CMPs or myeloid progenitor cells. Preferably, the rtTA gene or tTA gene operably linked to the third exogenous promoter is integrated into the genome of the CMP or myeloid progenitor cell.

From the viewpoint of enhancing proliferation ability, the cell 2 of the present invention may further have a nucleic acid encoding an expression suppressor nucleic acid (e.g., siRNA, shRNA, antisense nucleic acid) for the CDKN1A gene operably linked to a fifth exogenous promoter and/or a nucleic acid encoding an expression suppressor nucleic acid for the p53 gene operably linked to a sixth exogenous promoter. The explanation on the terms for the fifth exogenous promoter and sixth exogenous promoter follows the part described in Methodology 1. The nucleic acid encoding an expression suppressor nucleic acid for the CDKN1A gene operably linked to the fifth exogenous promoter and the nucleic acid encoding an expression suppressor nucleic acid for the p53 gene operably linked to the sixth exogenous promoter may be integrated into the genome of CMP or myeloid progenitor cells, or may be present in an expression vector introduced into CMPs or myeloid progenitor cells. Preferably, they are integrated into the genome of the CMP or myeloid progenitor cell.

In one embodiment, the cell 2 of the present invention is a CMP or a myeloid progenitor cell (e.g., GMP, macrophage progenitor cell, dendritic cell progenitor cell) having a MYC family gene (e.g., c-Myc) operably linked to the first exogenous promoter,
BMI1 gene operably linked to the second exogenous promoter, and BCL-XL gene operably linked to the fourth exogenous promoter. When a tetracycline-responsive promoter is used as at least one (preferably all) selected from the first, the second, and the fourth exogenous promoters, the cell of the present invention further optionally has rtTA gene or tTA gene operably linked to the third exogenous promoter.

In one embodiment, the cell 2 of the present invention is a CMP or a myeloid progenitor cell (e.g., GMP, macrophage progenitor cell, dendritic cell progenitor cell) having a MYC family gene operably linked to the first exogenous promoter,
BMI1 gene operably linked to the second exogenous promoter, BCL-XL gene operably linked to the fourth exogenous promoter, a nucleic acid encoding expression suppressor nucleic acid for CDKN1A gene operably linked to the fifth exogenous promoter, and
a nucleic acid encoding expression suppressor nucleic acid for p53 gene operably linked to the sixth exogenous promoter. When a tetracycline-responsive promoter is used as at least one (preferably all) selected from the first, the second, and the fourth exogenous promoters, the cell of the present invention further optionally has rtTA gene or tTA gene operably linked to the third exogenous promoter.

The cell 2 of the present invention can be obtained by the aforementioned method 2 of the present invention, or the production method 2 of the present invention.

In addition, the present invention provides a cell population containing the above-mentioned cell 2 of the present invention (to be referred to as the cell population 2 of the present invention). The cell population 2 abundantly contains the above-mentioned cell 2 of the present invention, and the ratio of the cell 2 of the present invention contained in the whole cell population 2 is, for example, not less than 10%, not less than 20%, not less than 30%, not less than 40%, not less than 50%, not less than 60%, not less than 70%, not less than 80%, not less than 90%, not less than 95% (e.g., 100%). Such cell population abundantly containing the cell 2 of the present invention can be obtained by extracting the cells of interest in a specific stage of differentiation (CMPs or myeloid progenitor cells (e.g., GMP, macrophage progenitor cell, dendritic cell progenitor cell)) from a cell population subjected to the above-mentioned method 2 of the present invention. In a preferred embodiment, the cell population 2 of the present invention abundantly contains the cell 2 of the present invention in a specific stage of differentiation. In one embodiment, the ratio of the cell 2 of the present invention contained in the whole cell population is (the cell is GMP) not less than 10%, not less than 20%, not less than 30%, not less than 40%, not less than 50%, not less than 60%, not less than 70%, not less than 80%, not less than 90%, not less than 95% (e.g., 100%). In one embodiment, the ratio of the cell 2 of the present invention contained in the whole cell population is (the cell is macrophage progenitor cell) not less than 10%, not less than 20%, not less than 30%, not less than 40%, not less than 50%, not less than 60%, not less than 70%, not less than 80%, not less than 90%, not less than 95% (e.g., 100%). In one embodiment, the ratio of the cell 2 of the present invention contained in the whole cell population is (the cell is dendritic cell progenitor cell) not less than 10%, not less than 20%, not less than 30%, not less than 40%, not less than 50%, not less than 60%, not less than 70%, not less than 80%, not less than 90%, not less than 95% (e.g., 100%). Such cell population abundantly containing the cell 2 of the present invention in a specific stage of differentiation can be obtained by isolating and extracting the cells of interest in a specific stage of differentiation from a cell population subjected to the above-mentioned method 2 of the present invention by using a cell sorter or the like and an antibody against a cell surface marker specifically expressed in the cell in the differentiation stage.

Furthermore, the present invention provides a cell preparation (to be referred to as the cell preparation 2 of the present invention) containing the above-mentioned cell population 2 of the present invention. The cell preparation 2 of the present invention can be prepared by, similar to Methodology 1, suspending the above-mentioned cell population 2 of the present invention in an appropriate physiological aqueous solution. In one embodiment, the cell preparation 2 of the present invention is a frozen cell preparation containing the above-mentioned frozen cell population 2 of the present invention.

Similar to Methodology 1, CMP-lineage differentiated cells can be obtained by differentiating the CMPs or myeloid progenitor cells obtained by the method 2 of the present invention or the production method 2 of the present invention (that is, the cell 2 of the present invention).

The proliferation promoting agent for CMPs or myeloid progenitor cells according to the present embodiment (to be referred to as proliferation promoting agent 2 of the present invention) contains, as an active ingredient, a molecule that forcibly expresses the BCL-XL gene. The proliferation promoting agent 2 of the present invention may further contain, as an active ingredient, a molecule that forcibly expresses the MYC family gene and the BMI1 gene. The proliferation promoting agent 2 of the present invention may further contain, as an active ingredient, a molecule that suppresses the expression of CDKN1A gene or p53 gene or the function of expression products thereof.

In addition, it is possible to prepare a pharmaceutical composition containing CMPs or myeloid progenitor cells obtained by the method 2 of the present invention or the production method 2 of the present invention (the cell 2 of the present invention), or CMP-lineage differentiated cells obtained by the aforementioned method for producing CMP-lineage differentiated cells, and use the composition for the treatment or prophylaxis of various diseases. Adjustment of the pharmaceutical composition and treatment or prophylaxis of diseases can be performed according to Methodology 1.

The definition of each term follows the part described in Methodology 1.

### (2) Methodology 3

In a further aspect, the present invention provides a method for improving proliferative property of CMPs or myeloid progenitor cells, including a step of suppressing the expression of CDKN1A gene and/or p53 gene or the function of expression products thereof in the CMPs or myeloid progenitor cells (hereinafter to be referred to as "the method 2 of the present invention"). The myeloid progenitor cell is preferably a GMP, a macrophage progenitor cell, a dendritic cell progenitor cell, or an erythrocyte progenitor cell. Suppressing the expression of the CDKN1A gene and/or p53 gene, or the function of expression products thereof, can be expected to improve the proliferative property of CMPs or myeloid progenitor cells and afford an immortalized cell line that proliferates infinitely.

It is preferable to suppress the expression of not only the CDKN1A gene but the p53 gene, or the function of expression products thereof.

Suppression of the expression of the above-mentioned respective genes or the function of expression products thereof can be performed according to the methods described in Methodology 1.

Suppression of the expression of the CDKN1A gene and/or the p53 gene is preferably performed, similar to Methodology 1, by introducing, into the cells, expression vectors expressing the expression suppressor nucleic acids (e.g., siRNA, shRNA, antisense nucleic acid) for the respective genes. CMP or myeloid progenitor cells may be transfected with an expression vector (e.g., viral vector) of an expression suppressor nucleic acid for the desired gene (e.g., CDKN1A gene, p53 gene), or CMPs or myeloid progenitor cells may be induced from pluripotent stem cells (e.g., ES cells, iPS cells), hematopoietic progenitor cells, or hematopoietic endothelial cells that have been incorporated in advance with expression cassettes of an expression suppressor nucleic acid for the desired gene (e.g., CDKN1A gene, p53 gene), and the expression suppressor nucleic acid may be forcibly expressed at this stage. Alternatively, CMPs or myeloid progenitor cells may be induced by differentiating pluripotent stem cells, hematopoietic progenitor cells, or hematopoietic endothelial cells that have been incorporated in advance with expression cassettes for the desired gene (e.g., CDKN1A gene, p53 gene), and in which the said expression suppressor nucleic acid is forcibly expressed. When the expression suppressor nucleic acid is expressed using an expression vector, a nucleic acid encoding (e.g., DNA) the expression suppressor nucleic acid can be linked downstream of an appropriate promoter, inserted into the expression vector, and then introduced into the cell to express the expression suppressor nucleic acid of interest. The promoter can be an exogenous promoter. The exogenous promoter may be a constitutive promoter or a regulatable promoter, preferably a constitutive promoter. The nucleic acid encoding the expression suppressor nucleic acid for the CDKN1A gene and the nucleic acid encoding the expression suppressor nucleic acid for the p53 gene may be inserted into separate expression vectors, or may be inserted into the same expression vector.

The method 3 of the present invention may further include a step of extracting (isolating or purifying) cells (CMPs or myeloid progenitor cells) at a specific desired stage of differentiation. The cells (CMPs or myeloid progenitor cells) at a specific stage of differentiation may be extracted before or after suppressing the expression of CDKN1A gene and/or p53 gene or the function of expression products thereof. In one embodiment, this extraction step is performed before suppressing the expression of CDKN1A gene and/or p53 gene or the function of expression products thereof, and the expression of CDKN1A gene and/or p53 gene or the function of expression products thereof is suppressed in the extracted cells (CMPs or myeloid progenitor cells) at a specific stage of differentiation. In another embodiment, a cell population containing cells (CMPs or myeloid progenitor cells) at a specific desired stage of differentiation in which the expression of CDKN1A gene and/or p53 gene or the function of expression products thereof has been suppressed is prepared, and then the cells (CMPs or myeloid progenitor cells) at a specific desired stage of differentiation are extracted from the cell population. The expression of CDKN1A gene and/or p53 gene or the function of expression products thereof may continuously be suppressed in these extracted cells at a specific stage of differentiation (CMPs or myeloid progenitor cells). By extracting the cells of interest and applying the method 3 of the present invention to the extracted cells, or by extracting the cells of interest from a cell population to which the method 3 of the present invention has been applied and continuously culturing the cells, the cell type of interest can be efficiently proliferated. From the aspect of efficiently improving the proliferative property of the extracted cell type, the cell to be extracted is preferably only CMP or only a single type of cell of myeloid progenitor cell, or may be a cell population of a mixture of two or more types of these cells. The cell to be extracted is preferably CMP, GMP, a macrophage progenitor cell, a dendritic cell progenitor cell, or an erythrocyte progenitor cell. Extraction of the cells of interest can be performed by the methods described in Methodology 1. The cells of interest may be isolated such that the percentage of the cells of interest in the cell population after the extraction operation is, for example, not less than 10%, not less than 20%, not less than 30%, not less than 40%, not less than 50%, not less than 60%, not less than 70%, not less than 80%, not less than 90%, not less than 95% (e.g., 100%). Single cells of the cells of interest may be isolated.

The method 3 of the present invention may further include a step of forcibly expressing a MYC family gene (preferably c-Myc) and BMI1 gene in CMPs or myeloid progenitor cells. By expressing the MYC family gene (preferably c-Myc) and the BMI1 gene in addition to the suppression of the expression of the CDKN1A gene and/or p53 gene, or the function of expression products thereof, further promotion of proliferation of CMPs or myeloid progenitor cells can be expected. The period of the above-mentioned forced expression of MYC family gene (preferably c-Myc) and BMI1 gene can be appropriately determined by those of ordinary skill in the art.

The forced expression of the MYC family gene and BMI1 gene may be performed simultaneously or successively with the suppression of the expression of the CDKN1A gene and/or p53 gene, or the function of expression products thereof. For example, the MYC family gene and the BMI1 gene may be forcibly expressed, and the expression of the CDKN1A gene and/or p53 gene or the function of expression products thereof may be successively suppressed to obtain CMPs or myeloid progenitor cells with improved proliferation potency. In addition, CMPs or myeloid progenitor cells with improved proliferation potency can also be obtained by simultaneously performing forced expression of the MYC family gene and the BMI1 gene, and suppression of the expression of the CDKN1A gene and/or p53 gene, or the function of expression products thereof.

The method 3 of the present invention may further include a step of forcing BCL-XL gene in CMPs or myeloid progenitor cells. By expressing the BCL-XL gene in addition to the suppression of the expression of the CDKN1A gene and/or p53 gene, or the function of expression products thereof, further promotion of proliferation of CMPs or myeloid progenitor cells can be expected. The period of the above-mentioned forced expression of the BCL-XL gene can be appropriately determined by those of ordinary skill in the art.

The forced expression of the BCL-XL gene may be performed simultaneously or successively with the suppression of the expression of the CDKN1A gene and/or p53 gene, or the function of expression products thereof. For example, the BCL-XL gene may be forcibly expressed and then the expression of the CDKN1A gene and/or p53 gene or the function of expression products thereof is suppressed to obtain CMPs or myeloid progenitor cells with improved proliferation potency. In addition, CMPs or myeloid progenitor cells with improved proliferation potency can also be obtained by simultaneously performing forced expression of the BCL-XL gene, and the suppression of the expression of the CDKN1A gene and/or p53 gene, or the function of expression products thereof.

In one embodiment, the method 3 of the present invention may further include a step of forcibly expressing the MYC family gene (preferably c-Myc gene), BMI1 gene, and BCL-XL gene in the CMPs or myeloid progenitor cells. In the present embodiment, the suppression of the expression of the CDKN1A gene or p53 gene or the function of expression products thereof may be performed simultaneously with the forced expression of any of the MYC family gene, BMI1 gene, and BCL-XL gene. Preferably, the expression of the CDKN1A gene or p53 gene or the function of expression products thereof is suppressed simultaneously with or after the forced expression of the BCL-XL gene. In one embodiment, in CMPs or myeloid progenitor cell, the expression of the CDKN1A gene and/or p53 gene or the function of expression products thereof is suppressed after a decrease is confirmed in the cell proliferation of CMPs or myeloid progenitor cells in which the MYC family gene (preferably c-Myc gene) and the BMI1 gene have been forcibly expressed. In one embodiment, in CMPs or myeloid progenitor cells, forced expression of the BCL-XL gene and suppression of the expression of the CDKN1A gene or p53 gene or the function of expression products thereof are performed after a decrease is confirmed in the cell proliferation of CMPs or myeloid progenitor cells in which the MYC family gene (preferably c-Myc gene) and the BMI1 gene have been forcibly expressed. In one embodiment, the cell proliferation rate at a certain time point is compared to the most recent cell proliferation rate (for example, when cell proliferation is confirmed every week, the cell proliferation rate of a certain week is compared with the proliferation rate of one week before), and it can be performed after confirmation of the state in which the proliferation rate has decreased to 1/2 or less. In one embodiment, in CMPs or myeloid progenitor cells, the MYC family gene (e.g., c-Myc gene) and the BMI1 gene are forcibly expressed and, in parallel, the expression of the CDKN1A gene and/or p53 gene or the function of expression products thereof is suppressed. In one embodiment, in CMPs or myeloid progenitor cells, the MYC family gene (e.g., c-Myc gene), the BMI1 gene, and the BCL-XL gene are forcibly expressed and, in parallel, the expression of CDKN1A gene and/or p53 gene or the function of expression products thereof is suppressed.

The MYC family gene, BMI1 gene, and BCL-XL gene promote proliferation of CMPs or myeloid progenitor cells, but can inhibit terminal differentiation of CMP-lineage differentiated cells (e.g., macrophage, dendritic cell, neutrophil, erythrocyte). Thus, expression of these genes may be suppressed before entering the terminal differentiation step. Suppressing the expression of these genes in CMPs or myeloid progenitor cells facilitates the induction of functional, more mature CMP-lineage differentiated cells (e.g., macrophage, dendritic cell, neutrophil, erythrocyte).

Genes such as the MYC family gene, BMI1 gene, and BCL-XL gene, can be forcibly expressed in cells according to the methods described in Methodology 1 and Methodology 2. Suppression of the expression of the MYC family gene, BMI1 gene, BCL-XL gene, and the like in cells may be performed according to the methods described in Methodology 1 and Methodology 2.

The present invention also provides a method for producing CMPs or myeloid progenitor cells, including a step of culturing CMPs or myeloid progenitor cells obtained in the above-mentioned method 3 of the present invention (culture step) (hereinafter to be referred to as the production method 2 of the present invention).

The culture conditions for CMPs or myeloid progenitor cells are as described in Methodology 1. As described in Methodology 1, a combination of cytokines suitable for cell proliferation can be added to the medium, depending on the type of CMPs or myeloid progenitor cells to be cultured.

The present invention provides a CMP or a myeloid progenitor cell having a nucleic acid encoding an expression suppressor nucleic acid (e.g., siRNA, shRNA, antisense nucleic acid) for the CDKN1A gene operably linked to a fifth exogenous promoter and/or a nucleic acid encoding an expression suppressor nucleic acid for the p53 gene operably linked to a sixth exogenous promoter (hereinafter the cell 3 of the present invention). The myeloid progenitor cell is preferably a GMP, a macrophage progenitor cell, a dendritic cell progenitor cell, or an erythrocyte progenitor cell. The explanation on the terms for the fifth exogenous promoter and sixth exogenous promoter follows the part described in Methodology 1. The nucleic acid encoding an expression suppressor nucleic acid for the CDKN1A gene operably linked to the fifth exogenous promoter and/or the nucleic acid encoding an expression suppressor nucleic acid for the p53 gene operably linked to the sixth exogenous promoter may be integrated into the genome of CMP or myeloid progenitor cell, or may also be present in an expression vector introduced into CMPs or myeloid progenitor cells. Preferably, they are integrated into the genome of the CMP or myeloid progenitor cell.

From the viewpoint of enhancing proliferation ability, the cell 3 of the present invention may further have a MYC family gene operably linked to the first exogenous promoter and BMI1 gene operably linked to the second exogenous promoter. The explanation on the terms for the first exogenous promoter and the second exogenous promoter follows the part described in Methodology 1. The MYC family gene operably linked to the first exogenous promoter and the BMI1 gene operably linked to the second exogenous promoter may be integrated into the genome of the CMP or myeloid progenitor cells, or they may be present in an expression vector introduced into the CMPs or myeloid progenitor cells. Preferably, the MYC family gene operably linked to the first exogenous promoter and the BMI1 gene operably linked to the second exogenous promoter are integrated into the genome of the CMP or myeloid progenitor cell.

From the viewpoint of enhancing proliferation ability, the cell 3 of the present invention may further have BCL-XL gene operably linked to the fourth exogenous promoter. The explanation on the terms for the fourth exogenous promoter follows the part described in Methodology 1. The BCL-XL gene operably linked to the fourth exogenous promoter may be integrated into the genome of the CMP or myeloid progenitor cell or it may be present in an expression vector introduced into the CMPs or myeloid progenitor cells. Preferably, the BCL-XL gene operably linked to the fourth exogenous promoter operably linked to the BCL-XL gene is integrated into the genome of the CMP or myeloid progenitor cell.

The type of the first, the second, and the fourth exogenous promoters may be the same as or different from that of the fourth exogenous promoter, and they are preferably the same type of promoters. By using the same type of promoters, the MYC family gene, BMI1 gene, and BCL-XL gene can be expressed synchronously, and the expression thereof can also be suppressed synchronously. The first exogenous promoter, the second exogenous promoter, and the fourth exogenous promoter are preferably the same regulatable promoters (e.g., drug-responsive promoters), more preferably all tetracycline-responsive promoters.

When using a tetracycline-responsive promoter as at least one of the first, the second, and the fourth exogenous promoters, in order to enable tetracycline-dependent expression control, the cell of the present invention preferably further has an rtTA gene or tTA gene operably linked to a third exogenous promoter. The third exogenous promoter may be a constitutive promoter or regulatable promoter, preferably a constitutive promoter. The rtTA gene or tTA gene operably linked to the third exogenous promoter may be integrated into the genome of the CMP or myeloid progenitor cell, or may be present in an expression vector introduced into the genome of CMPs or myeloid progenitor cells. Preferably, the rtTA gene or tTA gene operably linked to the third exogenous promoter is integrated into the genome of the CMP or myeloid progenitor cell.

In one embodiment, the cell 3 of the present invention is a CMP or a myeloid progenitor cell (e.g., CMP, GMP, macrophage progenitor cell, dendritic cell progenitor cell, erythrocyte progenitor cell) having
a MYC family gene (e.g., c-Myc) operably linked to the first exogenous promoter,
BMI1 gene operably linked to the second exogenous promoter, a nucleic acid encoding expression suppressor nucleic acid for CDKN1A gene operably linked to the fifth exogenous promoter, and
a nucleic acid encoding expression suppressor nucleic acid for p53 gene operably linked to the sixth exogenous promoter. When a tetracycline-responsive promoter is used as at least one (preferably all) selected from the first and the second exogenous promoters, the cell of the present invention further optionally has rtTA gene or tTA gene operably linked to the third exogenous promoter.

In one embodiment, the cell 3 of the present invention is a CMP or a myeloid progenitor cell (e.g., CMP, GMP, macrophage progenitor cell, dendritic cell progenitor cell, erythrocyte progenitor cell) having
a MYC family gene operably linked to the first exogenous promoter,
BMI1 gene operably linked to the second exogenous promoter, BCL-XL gene operably linked to the fourth exogenous promoter, a nucleic acid encoding expression suppressor nucleic acid for CDKN1A gene operably linked to the fifth exogenous promoter, and
a nucleic acid encoding expression suppressor nucleic acid for p53 gene operably linked to the sixth exogenous promoter. When a tetracycline-responsive promoter is used as at least one (preferably all) selected from the first, the second, and the fourth exogenous promoters, the cell of the present invention further optionally has rtTA gene or tTA gene operably linked to the third exogenous promoter.

The cell 3 of the present invention can be obtained by the aforementioned method 3 of the present invention, or the production method 3 of the present invention.

In addition, the present invention provides a cell population containing the above-mentioned cell 3 of the present invention (to be referred to as the cell population 3 of the present invention). The cell population 3 abundantly contains the above-mentioned cell 3 of the present invention, and the ratio of the cell 3 of the present invention contained in the whole cell population 3 is, for example, not less than 10%, not less than 20%, not less than 30%, not less than 40%, not less than 50%, not less than 60%, not less than 70%, not less than 80%, not less than 90%, not less than 95% (e.g., 100%). Such cell population abundantly containing the cell 3 of the present invention can be obtained by extracting the cells of interest in a specific stage of differentiation (CMPs or myeloid progenitor cells (e.g., GMP, macrophage progenitor cell, dendritic cell progenitor cell)) from a cell population subjected to the above-mentioned method 3 of the present invention. In a preferred embodiment, the cell population 3 of the present invention abundantly contains the cell 3 of the present invention in a specific stage of differentiation. In one embodiment, the ratio of the cell 3 of the present invention contained in the whole cell population is (the cell is GMP) not less than 10%, not less than 20%, not less than 30%, not less than 40%, not less than 50%, not less than 60%, not less than 70%, not less than 80%, not less than 90%, not less than 95% (e.g., 100%). In one embodiment, the ratio of the cell 3 of the present invention contained in the whole cell population is (the cell is macrophage progenitor cell) not less than 10%, not less than 20%, not less than 30%, not less than 40%, not less than 50%, not less than 60%, not less than 70%, not less than 80%, not less than 90%, not less than 95% (e.g., 100%). In one embodiment, the ratio of the cell 3 of the present invention contained in the whole cell population is (the cell is dendritic cell progenitor cell) not less than 10%, not less than 20%, not less than 30%, not less than 40%, not less than 50%, not less than 60%, not less than 70%, not less than 80%, not less than 90%, not less than 95% (e.g., 100%). In one embodiment, the ratio of the cell 3 of the present invention contained in the whole cell population is (the cell is erythrocyte progenitor cell) not less than 10%, not less than 20%, not less than 30%, not less than 40%, not less than 50%, not less than 60%, not less than 70%, not less than 80%, not less than 90%, not less than 95% (e.g., 100%). Such cell population abundantly containing the cell 3 of the present invention in a specific stage of differentiation can be obtained by isolating and extracting the cells of interest in a specific stage of differentiation from a cell population subjected to the above-mentioned method 3 of the present invention by using a cell sorter or the like and an antibody against a cell surface marker specifically expressed in the cell in the differentiation stage.

Furthermore, the present invention provides a cell preparation (to be referred to as the cell preparation 3 of the present invention) containing the above-mentioned cell population 3 of the present invention. The cell preparation 3 of the present invention can be prepared by, similar to Methodology 1, suspending the above-mentioned cell population 3 of the present invention in an appropriate physiological aqueous solution. In one embodiment, the cell preparation 3 of the present invention is a frozen cell preparation containing the above-mentioned frozen cell population 3 of the present invention.

Similar to Methodology 1, CMP-lineage differentiated cells can be obtained by differentiating the CMP or myeloid progenitor cell obtained by the method 3 of the present invention or the production method 3 of the present invention (that is, the cell 3 of the present invention).

The proliferation promoting agent for CMPs or myeloid progenitor cells according to the present embodiment (to be referred to as proliferation promoting agent 3 of the present invention) contains, as an active ingredient, a molecule that suppresses the expression of CDKN1A gene and/or p53 gene or the function of expression products thereof. The proliferation promoting agent 3 of the present invention preferably contains, as an active ingredient, an expression vector capable of expressing an expression suppressor nucleic acid (e.g., siRNA, shRNA, antisense nucleic acid) capable of specifically suppressing the expression of the CDKN1A gene or an expression suppressor nucleic acid of these, and/or an expression suppressor nucleic acid capable of specifically suppressing the expression of the p53 gene, or an expression vector capable of expressing these expression suppressor nucleic acids thereof. The proliferation promoting agent 3 of the present invention may further contain, as an active ingredient, a molecule that forcibly expresses the MYC family gene and the BMI1 gene. The proliferation promoting agent 3 of the present invention may further contain, as an active ingredient, a molecule that forcibly expresses the BCL-XL gene.

In addition, it is possible to prepare a pharmaceutical composition containing CMPs or myeloid progenitor cells obtained by the method 3 of the present invention or the production method 3 of the present invention (the cell 3 of the present invention), or CMP-lineage differentiated cell obtained by the aforementioned method for producing CMP-lineage differentiated cell, and use the composition for the treatment or prophylaxis of various diseases. Preparation of the pharmaceutical composition and treatment or prophylaxis of diseases can be performed according to Methodology 1.

The definition of each term follows the part described in Methodology 1.

The present invention is explained in detail in the following by referring to Examples, which are not to be construed as limitative. Those skilled in the art can modify the present invention in various ways without departing from the meaning of the present invention, and such modifications are also included in the scope of the present invention.

### [Example]

### Example 1-1: Promotion of CMP cell line proliferation

Human iPS cells were cultured for 14 days according to the method shown in Fig. 1, differentiated into blood progenitor cells, and CD34⁺CD43⁺ cells were sorted by a cell sorter to obtain isolated blood progenitor cells. The obtained blood progenitor cells were treated as follows to establish CMP cell line having proliferation potency. First, into the isolated blood progenitor cells (1.0×10⁴ to 1.0×10⁵ cells) on day 14 of culture was introduced lentiviral vector that forces expression of c-MYC/BMI1 (MB) through doxycycline control and the cells were cultured in the presence of doxycycline in a medium containing GM-CSF (50 ng/ml), G-CSF (10 ng/ml), IL-3 (10 ng/ml), SCF (25 ng/ml), and TPO (5 ng/ml) to establish CMP (MB) with proliferation potency. The proliferated established cells were confirmed to be CMP by methylcellulose colony assay which showed induction of neutrophil, macrophage, erythroblast, and megakaryocyte from single cells (Takayama et al., Blood, 111(11):5298-5306, 2008).

On day 21 of culture, CMP (MB) was infected with a lentiviral vector that forcibly expresses BCL-XL under doxycycline control, and a lentiviral vector that continuously expresses shRNA for p21 and shRNA for p53, and the cells were further cultured in a medium containing GM-CSF, G-CSF, IL-3, SCF, and TPO in the presence of doxycycline. By this culture procedure, CMP (MBX) in which the BCL-XL gene was further introduced using a doxycycline-induced lentiviral vector in addition to MB introduction, CMP (MB-p21/p53_KD), which was further infected with continuously expressing sh p21/p53 lentiviral vector in addition to MB introduction, and CMP (MBX-p21/p53_KD) in which the BCL-XL gene was introduced using a doxycycline-induced lentiviral vector in addition to MB introduction which was infected with continuously expressing sh p21/p53 lentiviral vector were established. Since the established cells did not express markers for macrophage, MEP, and megakaryocyte precursor cell, they were considered to be a CMP single population.

The results of counting the number of cells on the 14th, 31st, and 43rd days for each CMP are shown in Fig. 2. Forced expression of c-MYC and BMI1 resulted in favorable proliferation of CMP. Forced expression of BCL-XL promoted cell proliferation. Cell proliferation was also promoted by knockdown of p21 and p53. Using a similar method, CMP with proliferative property was successfully established from seven different iPS cell lines.

### Example 1-2: Differentiation induction into erythroblast, macrophage and neutrophil

After removing doxycycline from the culture medium of each cell obtained in Example 1-1 and suppressing the expression of the three factors: c-MYC/BMI1/BCL-XL, the cells were cultured in the presence of cytokines of G-CSF, SCF, TPO, EPO, and IL3, and on day 7, erythroblasts, macrophages, and neutrophils differentiated from the CMP cell line were analyzed by FACS. As the antibody, CD43 antibody, CD33 antibody, CD14 antibody, CD11b antibody, GPA (Glycophorin A) antibody (BioLegend, catalog No.: 306612), and APC anti-human CD41 Antibody (BioLegend, catalog No.: 303710) were used. The results are shown in Fig. 3. Differentiation into megakaryocytes was also confirmed. Terminal differentiation into three major myeloid lineages was confirmed by doxycycline removal (suppression of c-MYC/BMI1/BCL-XL expression). These results suggest that the cells obtained in Example 1-1 are CMP and that terminal differentiation into myeloid cells is promoted by suppressing the expression of c-MYC/BMI1/BCL-XL.

### Example 1-3: Differentiation induction into macrophage and neutrophil

After removing doxycycline from the culture medium of each cell obtained in Example 1-1 and suppressing the expression of the three factors MYC/BMI1/BCL-XL, the cells were cultured in the presence of cytokines of GM-CSF, G-CSF, SCF, TPO, and IL3, and on day 7, erythroblasts, macrophages, and neutrophils differentiated from the CMP cell line were analyzed by FACS. As the antibody, CD16 antibody, CD14 antibody, CD11b antibody, and CD11c antibody were used. The results are shown in Fig. 4. Macrophages were induced from both CMPs of MBX and MBX-p21/p53KD. The percentage of cells differentiated into macrophages was higher in MBX-p21/p53KD than in MBX, suggesting that p21/p53KD may promote differentiation into macrophages.

### Example 2-1: PiggyBac System

BMI1 IRESS c-MYC was introduced downstream of the tetracycline response element (TRE) in the PiggyBac vector to create a vector in which sh p53 and sh p21 were introduced downstream of the H1 promoter (pb BMI1 IRESS c-MYC-rtTA sh p53 sh p21) (see Fig. 5(A)). BCL-XL was introduced downstream of TRE in the PiggyBac vector. In this vector, the rtTA 2A puromycin resistance gene is expressed downstream of the Ubic promoter (pb BclXL). The PiggyBac vector used was provided by Associate Professor Knut Woltjen of the Center for iPS Cell Research and Application, Kyoto University.

After introducing the above-mentioned vector into each cell line of 1383D10 (iPS cell), KhES3, and KthES14 by lipofection, the introduced cells were selected with puromycin. Thereafter, each cell was differentiated into blood progenitor cells and isolated in 14 days, and then cultured under the conditions shown in Fig. 5(B) to obtain each cell line of macrophage cell line and erythrocyte cell line.

More specifically, when preparing macrophage cell lines, blood progenitor cells were cultured for 7 days in the presence of SCF (50 ng/ml), M-CSF (20 ng/ml), IL1β (10 ng/ml), and Doxcycline (1 µg/ml), and CX3CR1-positive CD14-positive cells were isolated using a cell sorter. The isolated CX3CR1-positive CD14-positive cells were successively cultured in the presence of SCF (50 ng/ml), M-CSF (20 ng/ml), IL1β (10 ng/ml), and Doxcycline (1 µg/ml) to obtain a macrophage cell line.

When preparing erythrocyte cell lines, blood progenitor cells were cultured for 7 days in the presence of SCF (50 ng/ml), EPO (3 U/ml), and Doxcycline (1 µg/ml), and CD71-positive CD235ab-positive cells were isolated using a cell sorter. The isolated CD71-positive CD235ab-positive cells were successively cultured in the presence of SCF (50 ng/ml), EPO (3 U/ml) and Doxcycline (1 µg/ml) to obtain an erythrocyte cell line.

The proliferation curve of each macrophage cell line is shown in Fig. 6. As the cell number, CX3CR1-positive cells were counted.

### Example 2-2: Macrophage cell line

The 1383D10-derived macrophage cell line obtained in Example 2-1 was stained with CD13, CD14, CD33, CD43, and HLA-DR and analyzed by FACS. As the antibody, CD13 antibody, CD14 antibody, CD33 antibody, CD43 antibody, and HLA-DR antibody were used. The results are shown in Fig. 7. The cells were confirmed to be positive for macrophage markers: CD13 and CD14.

### Example 2-3: Macrophage cell surface marker

After sorting each macrophage cell line obtained in Example 2-1 using the macrophage marker: CX3CR1, macrophage cell surface markers were analyzed by FACS during gene expression (Dox on) and gene expression suppression (Dox off). In Dox on, the cells were cultured in the presence of SCF (50 ng/ml), M-CSF (20 ng/ml), IL1β (10 ng/ml), and Doxcycline (1 µg/ml), and in Dox off, the cells were cultured in the presence of M-CSF (20 ng/ml) for 3 days (see the upper part of Fig. 16). The results of gene expression (Dox on) are shown in the lower part of Fig. 8, and the results of gene expression suppression (Dox off) are shown in Fig. 9. In Dox off, CD14, CD163, and CD80 were highly expressed compared to Dox on. However, the expression of CD11b was low.

### Example 2-4: M1 type or M2 type

Whether each macrophage cell line obtained in Example 2-1 was M1 type or M2 type was analyzed by FACS. When Dox off cells were stained with CD32, which is an M1 type marker, and Cd163, which is an M2 type marker, both were positive and could not be identified by FACS (see Fig. 10).

### Example 2-5: CD11b-positive cell

Doxycycline was removed from the culture medium of each macrophage cell line obtained in Example 2-1, and the cell lines cultured on Matrigel in the presence of M-CSF during Dox off were stained with CD11b, whereby CD11b-positive cells were obtained. The results are shown in Fig. 11. It was found that a macrophage marker: CD11b was expressed by changing the culture environment (culturing on Matrigel).

### Example 2-6: Phagocytosis ability

The phagocytosis ability of each macrophage cell line obtained in Example 2-1 was examined. Doxycycline was removed from the culture medium of each cell, and a fluorescently labeled yeast cell wall peptide was added to the cell line on the 1st, 2nd, and 3rd days after Dox on and Dox off, followed by FACS analysis. The results are shown in Fig. 12. Phagocytosis ability was low in Dox ON. On the other hand, phagocytosis ability was confirmed from the first day in Dox off.

### Example 2-7: Phagocytosis ability of β-amiloid

The β-amiloid phagocytosis ability of each macrophage cell line obtained in Example 2-1 was examined. Doxycycline was removed from the culture medium of each cell, and fluorescently labeled oligomer β-amiloid was added to the cell line on the 5th day after Dox on and Dox off, followed by FACS analysis. The results are shown in Fig. 13. Phagocytosis ability was low in Dox ON. On the other hand, phagocytosis ability was confirmed in Dox off.

### Example 2-8: Erythrocyte line

In Example 2-1, blood progenitor cells derived from khES3 (ES cell) and 1383D10 (iPS cell) were cultured under the conditions of SCF (50 ng/ml), EPO (3 U/ml), and Dox (1 µg/ml). For the obtained immortalized erythrocyte line, the number of cells was counted with Gly-A positive cells (see Fig. 14).

### Example 3-1: PiggyBac System

In the same manner as in Example 2-1, on the 7th day of blood cell differentiation, five ES cell lines and five iPS cell lines were cultured under the conditions shown in Fig. 15 to obtain respective cell lines of macrophage cell line, dendritic cell line, and erythrocyte line.

More specifically, when preparing macrophage cell lines, hematopoietic endothelial cells were cultured for 7 days in the presence of SCF (50 ng/ml), M-CSF (20 ng/ml), IL1β (10 ng/ml), Doxcycline (1 µg/ml), and then CX3CR1-positive CD14-positive cells were isolated using a cell sorter. The isolated CX3CR1-positive CD14-positive cells were successively cultured in the presence of SCF (50 ng/ml), M-CSF (20 ng/ml), IL1β (10 ng/ml), and Doxcycline (1 µg/ml) to obtain a macrophage cell line.

When preparing a dendritic cell line, hematopoietic endothelial cells were cultured for 7 days in the presence of SCF (50 ng/ml), M-CSF (20 ng/ml), GM-CSF (20 µg/ml), and Doxcycline (1 µg/ml), and then CD209-positive cells were isolated using a cell sorter. The isolated CD209-positive cells were subsequently cultured in the presence of SCF (50 ng/ml), M-CSF (20 ng/ml), GM-CSF (20 µg/ml), and Doxcycline (1 µg/ml) to obtain a dendritic cell line.

When preparing erythrocyte cell lines, hematopoietic endothelial cells were cultured for 7 days in the presence of SCF (50 ng/ml), EPO (3 U/ml), and Doxcycline (1 µg/ml), and then CD71-positive CD235ab-positive cells were isolated using a cell sorter. The isolated CD71-positive CD235ab-positive cells were successively cultured in the presence of SCF (50 ng/ml), EPO (3 U/ml), and Doxcycline (1 µg/ml) to obtain an erythrocyte line.

A proliferation curve of each macrophage cell line having proliferation potency which was obtained by the culture under conditions of SCF (50 ng/ml), M-CSF (20 µg/ml), IL1β (10 ng/ml), and Dox (1 µg/ml) is shown in Fig. 16. The number of cells was determined by counting CX3CR1-positive cells.

A proliferation curve of each dendritic cell line having proliferation potency which was obtained by the culture under conditions of SCF (50 ng/ml), M-CSF (20 µg/ml), GM-CSF (20 µg/ml), and Dox (1 µg/ml) is shown in Fig. 17. The number of cells was determined by counting CD209-positive cells.

### Example 3-2: Macrophage cell surface marker

After sorting each macrophage cell line obtained in Example 3-1 using the macrophage marker: CX3CR1, macrophage cell surface markers were analyzed by FACS during gene expression (Dox on) and gene expression suppression (Dox off). In Dox on, the cells were cultured in the presence of SCF (50 ng/ml), M-CSF (20 ng/ml), IL1β (10 ng/ml), and Doxcycline (1 µg/ml), and in Dox off, the cells were cultured in the presence of M-CSF (20 ng/ml) for 3 days (see the upper part of Fig. 18). The results of gene expression (Dox on) are shown in the lower part of Fig. 18, and the results of gene expression suppression (Dox off) are shown in Fig. 19. In Dox off, CD14, CD163, and CD80 were highly expressed compared to Dox on. However, the expression of CD11b was low.

### Example 3-3: Phagocytosis ability of β-amiloid

The β-amiloid phagocytosis ability of each macrophage cell line obtained in Example 3-1 was examined. Doxycycline was removed from the culture medium of each cell, and fluorescently labeled oligomer β-amiloid was added to the cell line in Dox on and on the 3rd day after Dox off, followed by FACS analysis. The results are shown in Fig. 20. Phagocytosis ability was low in Dox ON. On the other hand, phagocytosis ability was confirmed in Dox off.

### Example 3-4: Dendritic cell surface marker

After sorting each dendritic cell line obtained in Example 3-1 using the dendritic cell marker CD209, dendritic cell surface markers were analyzed by FACS during gene expression (Dox on) and gene expression suppression (Dox off). In Dox on, the cells were cultured in the presence of SCF (50 ng/ml), M-CSF (20 ng/ml), GM-CSF (20 ng/ml), and Doxcycline (1 µg/ml), and in Dox off, the cells were culture in the presence of GM-CSF (20 ng/ml) for 5 days (see the upper part of Fig. 20). The results of gene expression (Dox on) and the results of gene expression suppression (Dox off) are respectively shown in Figs. 21 to 23. In Dox off, CD11c, CD209, and CD80 were highly expressed compared to Dox on. In addition, some cells expressed CDIa and CD1c, which are receptors that present bacterial lipid molecules to T cells as antigens when Dox off.

### Example 3-5: Erythrocyte line

In Example 3-1, blood progenitor cells derived from khES3 (ES cell) and kthES14 (ES cell) were cultured under the conditions of SCF (50 ng/ml), EPO (3 U/ml), and Dox (1 µg/ml). For the obtained erythrocyte line with proliferation potency, the number of cells was counted with Gly-A positive cells (see Fig. 24).

## Claims

1. A method for improving proliferative property of a common myeloid progenitor cell (CMP) or a myeloid progenitor cell, comprising a step of forcibly expressing a MYC family gene and BMI1 gene in any cell in the process of differentiation from a hematopoietic progenitor cell into a myeloid progenitor cell, wherein
the myeloid progenitor cell is a progenitor cell of macrophage, dendritic cell, granulocyte, erythroblast, or erythrocyte.

2. The method according to claim 1, further comprising a step of extracting the CMP or myeloid progenitor cell.

3. The method according to claim 1 or 2, further comprising a step of suppressing the expression of the MYC family gene and the BMI1 gene, or the function of an expression product thereof in the CMP or myeloid progenitor cell.

4. The method according to any one of claims 1 to 3, further comprising a step of forcibly expressing BCL-XL gene in the CMP or myeloid progenitor cell.

5. The method according to claim 4, further comprising a step of suppressing the expression of the BCL-XL gene, or the function of an expression product thereof in the CMP or myeloid progenitor cell.

6. The method according to any one of claims 1 to 5, further comprising a step of suppressing the expression of at least one of CDKN1A gene and p53 gene, or the function of an expression product thereof, in the CMP or myeloid progenitor cell.

7. A method for producing a CMP or a myeloid progenitor cell, comprising a step of culturing the CMP or myeloid progenitor cell obtained by the method according to any one of claims 1 to 6.

8. A method for producing a CMP-lineage differentiated cell, comprising a step of differentiating the CMP or myeloid progenitor cell obtained by the method according to any one of claims 1 to 7.

9. The method according to claim 8, wherein the CMP-lineage differentiated cell is macrophage, dendritic cell, granulocyte, erythroblast, or erythrocyte.

10. A pharmaceutical composition comprising the CMP or myeloid progenitor cell obtained by the method according to any one of claims 1 to 7, or the CMP-lineage differentiated cell obtained by the method according to claim 8 or 9.

11. The CMP or myeloid progenitor cell obtained by the method according to any one of claims 1 to 7.

12. The CMP-lineage differentiated cell obtained by the method according to claim 8 or 9.

13. A proliferation promoting agent of a CMP or a myeloid progenitor cell, comprising
a molecule that forcibly expresses a MYC family gene and BMI1 gene as an active ingredient, wherein
the myeloid progenitor cell is a progenitor cell of macrophage, dendritic cell, granulocyte, erythroblast, or erythrocyte.

14. A method for producing macrophages, comprising the following steps:
1) a step of forcibly expressing a MYC family gene and BMI1 gene in any cells in the process of differentiation from hematopoietic progenitor cells into macrophage progenitor cells,
2) a step of culturing and proliferating the cells obtained in step 1,
3) a step of suppressing forced expression of the MYC family gene and the BMI1 gene in the cells obtained in step 2 and further culturing the cells under macrophage differentiation conditions to promote differentiation into and maturation of macrophages.

15. The method according to claim 14, wherein the step 1 further comprises forcibly expressing BCL-XL gene in any cells in the process of differentiation from hematopoietic progenitor cells into macrophage progenitor cells.

16. The method according to claim 15, wherein the step 3 further comprises suppressing forced expression of the BCL-XL gene in the cells obtained in step 2.

17. The method according to any one of claims 14 to 16, wherein the step 1 further comprises suppressing expression of CDKN1A gene and/or p53 gene, or the function of an expression product thereof in any cells in the process of differentiation from hematopoietic progenitor cells into macrophage progenitor cells.
